# EUROPEAN PATENT SPECIFICATION

(11) **EP 0 984 930 B1**
(45) Date of publication and mention of the grant of the patent: **06.04.2005**
(21) Application number: 98924746.5
(22) Date of filing: 07.05.1998
(51) Int. Cl.: C07D 209/34, C07D 209/42, C07D 211/02, C07D 307/02, C07D 403/06, C07D 409/06, C07D 405/06, A61K 31/40

(54) **2-INDOLINONE DERIVATIVES AS MODULATORS OF PROTEIN KINASE ACTIVITY**
2-INDOLINONDERIVATE ALS MODULATOREN DER PROTEINKINASE-ATIVITÄT
DERIVES DE 2-INDOLINONE UTILISES EN TANT QUE MODULATEURS DE L'ACTIVITE DE LA PROTEINE KINASE

(30) Priority: 07.05.1997 US 45838 P; 08.05.1997 US 46868 P; 11.06.1997 US 49324 P; 20.06.1997 US 50413 P; 20.06.1997 US 50412 P; 20.06.1997 US 50977 P; 19.09.1997 US 59381 P; 19.09.1997 US 59384 P; 19.09.1997 US 59336 P; 19.09.1997 US 59544 P; 19.09.1997 US 59677 P; 25.09.1997 US 59971 P; 26.09.1997 US 60194 P
(43) Date of publication of application: 15.03.2000
(73) Proprietor: Sugen, Inc., South San Francisco, CA 94080-4811 (US)
(72) Inventor: TANG, Peng, Cho, Moraga, CA 94556 (US); SUN, Li, Foster City, CA 94404 (US); McMAHON, Gerald, Kenwood, CA 95452 (US); SHAWVER, Laura, Kay, San Francisco, CA 94112 (US); HIRTH, Klaus, Peter, San Francisco, CA 94114 (US)
(74) Representative: Viering, Jentschura & Partner
(86) International application number: PCT/US1998/009017
(87) International publication number: WO 1998/050356

(56) References cited:
- WO-A-95/14667
- WO-A-96/00226
- WO-A-96/22976
- WO-A-96/40116
- WO-A-98/07695
- US-A- 5 397 787

## Description

### Introduction

The present invention relates to novel 2-indolinone derivatives, more particularly to substituted diazaindolinones, to pharmaceutical compositions comprising such compounds, and to the use of such compounds in the manufacture of medicaments for the treatment of disorders related to abnormal protein kinase ("PK") activity.

### Background Of The Invention

The following is offered as background information only and is not admitted to be prior art to the present invention.

PKs are enzymes that catalyze the phosphorylation of hydroxy groups on tyrosine, serine and threonine residues of proteins. The consequences of this seemingly simple activity are staggering; cell growth, differentiation and proliferation; i.e., virtually all aspects of cell life in one way or another depend on PK activity. Furthermore, abnormal PK activity has been related to a host of disorders, ranging from relatively non-life threatening diseases such as psorisasis to extremely virulent diseases such as glioblastoma (brain cancer).

The PKs can conveniently be broken down into two classes, the protein tyrosine kinases (PTKs) and the serine-threonine kinases (STKs).

One of the prime aspects of PTK activity is its involvement with growth factor receptors. Growth factor receptors are cell-surface proteins. When bound by a growth factor ligand, growth factor receptors are converted to an active form which interacts with proteins on the inner surface of a cell membrane. This leads to phosphorylation on tyrosine residues of the receptor and other proteins and to the formation inside the cell of complexes with a variety of cytoplasmic signaling molecules that, in turn, affect numerous cellular responses such as cell division (proliferation), cell differentiation, cell growth, expression of metabolic effects to the extracellular microenvironment, etc. For a more complete discussion, see Schlessinger and Ullrich, Neuron, 9:303-391 (1992).

Growth factor receptors with PK activity are known as receptor tyrosine kinases ("RTKs"). They comprise a large family of transmembrane receptors with diverse biological activity. At present, at least nineteen (19) distinct subfamilies of RTKs have been identified. An example of these is the subfamily designated the "HER" RTKs, which include EGFR (epithelial growth factor receptor), HER2, HER3 and HER4. These RTKs consist of an extracellular glycosylated ligand binding domain, a transmembrane domain and an intracellular cytoplasmic catalytic domain that can phosphorylate tyrosine residues on proteins.

Another RTK subfamily consists of insulin receptor. (IR), insulin-like growth factor I receptor (IGF-1R) and the insulin receptor related receptor (IRR). IR and IGF-1R interact with insulin, IGF-I and IGF-II to form a heterotetramer of two entirely extracellular glycosylated α subunits and two β subunits which cross the cell membrane and which contain the tyrosine kinase domain.

A third RTK subfamily is referred to as the platelet derived growth factor receptor ("PDGFR") group, which includes PDGFRα, PDGFRβ, CSFIR, c-kit and c-fms. These receptors consist of glycosylated extracellular domains composed of variable numbers of immunoglobin-like loops and an intracellular domain wherein the tyrosine kinase domains is interrupted by unrelated amino acid sequences.

Another group which, because of its similarity to the PDGFR subfamily, is sometimes subsumed in the later group is the fetus liver kinase ("flk") receptor subfamily. This group is believed to be made of up of kinase insert domain-receptor fetal liver kinase-1 (KDR/FLK-1), flk-1R, flk-4 and fms-like tyrosine kinase 1 (flt-1).

One further member of the tyrosine kinase growth factor receptor family is the fibroblast growth factor ("FGF") receptor group. This groups consists of four receptors, FGFR1-4, and seven ligands, FGF1-7. While not yet well defined, it appears that the receptors consist of a glycosylated extracellular domain containing a variable number of immunoglobin-like loops and an intracellular domain in which the PTK sequence is interrupted by regions of unrelated amino acid sequences.

A more complete listing of the known RTK subfamilies is described in Plowman et al., DN&P, 7(6):334-339 (1994).

In addition to the RTKs, there also exists a family of entirely intracellular PTKs called "non-receptor tyrosine kinases" or "cellular tyrosine kinases". This latter designation, abbreviated "CTK", will be used herein. CTKs do not contain extracellular and transmembrane domains. At present, over 24 CTKs in 11 subfamilies (Src, Frk, Btk, Csk, Abl, Zap70, Fes/Fps, Fak, Jak and Ack) have been identified. The Src subfamily appear so far to be the largest group of CTKs and includes Src, Yes, Fyn, Lyn, Lck, Blk, Hck, Fgr and Yrk. For a more detailed discussion of CTKs, see Bolen, Oncogene, 8:2025-2031 (1993).

The serine-threonine kinases or STKs, like the CTKs, are predominantly intracellular although there are a few receptor kinases of the STK type. STKs are the most common of the cytosolic kinases; i.e., kinases which perform their function in that part of the cytoplasm other than the cytoplasmic organelles and cytoskelton. The cytosol is the region within the cell where much of the cell's intermediary metabolic and biosynthetic activity occurs; e.g., it is in the cytosol that proteins are synthesized on ribosomes.

RTKs, CTKs and STKs have all been implicated in a host of pathogenic conditions including, significantly, large number of diverse cancers. Others pathogenic conditions which have been associated with PTKs include, without limitation, psoriasis, hepatic cirrhosis, diabetes, atherosclerosis, angiogenesis, restinosis, ocular diseases, rheumatoid arthritis and other inflammatory disorders, autoimmune disease and a variety of renal disorders.

With regard to cancer, two of the major hypotheses advanced to explain the excessive cellular, proliferation that drives tumor development relate to functions known to be PK regulated. That is, it has been suggested that malignant cell growth results from a breakdown in the mechanisms that control cell division and/or differentiation. It has been shown that the protein products of a number of proto-oncogenes are involved in the signal transduction pathways that regulate cell growth and differentiation. These protein products of proto-oncogenes include the extracellular growth factors, transmembrane growth factor PTK receptors (RTKs), cytoplasmic PTKs (CTKs) and cytosolic STKs, discussed above.

In view of the apparent link between PK-related cellular activities and a number of human disorders, it is no surprise that a great deal of effort is being expended in an attempt to identify ways to modulate PK activity. Some of these have involved biomimetic approaches using large molecules patterned on those involved in the actual cellular processes (e.g., mutant ligands (U.S. App. No. 4,966,849); soluble receptors and antibodies (App. No. WO 94/10202, Kendall and Thomas, Proc. Nat'l Acad. Sci., 90:10705-09 (1994), Kim, et al., Nature, 362:841-844 (1993)); RNA ligands (Jelinek, et al., Biochemistry, 33:10450-56); Takano, et al., Mol. Bio. Cell 4:358A (1993); Kinsella, et al., Exp. Cell Res. 199:56-62 (1992); Wright, et al., J. Cellular Phys., 152:448-57) and tyrosine kinase inhibitors (WO 94/03427; WO 92/21660; WO 91/15495; WO 94/14808; U.S. Pat. No. 5,330,992; Mariani, et al., Proc. Am. Assoc. Cancer Res., 35:2268 (1994)).

More recently, attempts have been made to identify small molecules which act as PK inhibitors. For example, bis- monocylic, bicyclic and heterocyclic aryl compounds (PCT WO 92/20642), vinylene-azaindole derivatives (PCT WO 94/14808) and 1-cyclopropyl-4-pyridylquinolones (U.S. Pat. No. 5,330,992) have been described as PTK inhibitors. Styryl compounds (U.S. Pat. No. 5,217,999), styryl-substituted pyridyl compounds (U.S. Pat. No. 5,302,606), quinazoline derivatives (EP App. No. 0 566 266 A1), selenaindoles and selenides (PCT WO 94/03427), tricyclic polyhydroxylic compounds (PCT WO 92/21660) and benzylphosphonic acid compounds (PCT WO 91/15495) have all been described as PTK inhibitors useful in the treatment of cancer.

Our own efforts to identify small organic molecules which modulate PK activity and which, therefore, should be useful in the treatment and prevention of disorders driven by abnormal PK activity, has led us to the discovery of a family of novel 2-indolinone derivatives which exhibit excellent PK modulating ability and which are the subject of this invention.

### Summary Of The Invention

The present invention relates generally to novel 2-indolinone derivatives and their salts which modulate the activity of receptor protein tyrosine kinases (RTK), non-receptor protein tyrosine kinases (CTK) and serine/threonine protein kinases (STK). In particular the invention relates to 3-methylidenyl substituted diaza-2-indolinones.

In addition the present invention relates to pharmacological compositions of the disclosed compounds and their physiologically acceptable salts and a pharmaceutically acceptable diluent or carrier.

The invention also relates to the use of such compounds in the manufacture of medicaments for the treatment of disorders related to abnormal protein kinase activity such as cancer, other proliferation disorders, diabetes, autoimmune disease, inflammatory disorders and angiogenesis.

### 1. The Compounds

In a first aspect the present invention features compounds having the general chemical structure: 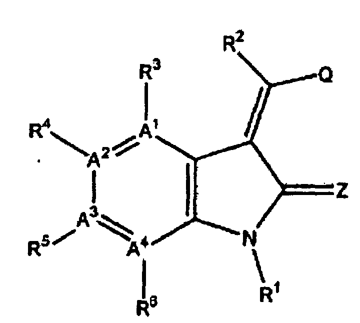 wherein,
A² and A⁴ are nitrogen and A¹ and A³ are carbon or A¹ and A³ are nitrogen and A² and A⁴ are carbon, it being understood that when A¹, A², A³ and A⁴ is nitrogen, R³, R⁴, R⁵ and R⁶, respectively does not exist;
R¹ is selected from the group consisting of hydrogen; C1-C10 alkyl, cycloalkyl, C2-C10 alkenyl, C2-C10 alkynyl, aryl, heteroaryl, trihalomethanecarbonyl, heteroalicyclic, hydroxy, C1-C10 alkoxy, C-carboxy, O-carboxy, C-amido, C-thioamido, guanyl, guanadinyl, ureidyl, sulfonyl, and trihalomethanesulfonyl;
R² is selected from the group consisting of hydrogen, C1-C10 alkyl, cycloalkyl, aryl, and halo;
R³, R⁴, R⁵ and R⁶ are independently selected from the group consisting of hydrogen, C1-C10 alkyl, trihalomethyl, cycloalkyl, C2-C10 alkenyl, C2-C10 alkynyl, aryl, heteroaryl, heteroalicyclic, hydroxy, thiohydroxy, C1-C10 alkoxy, aryloxy, thiohydroxy, C1-C10 thioalkoxy, thioaryloxy, sulfinyl, sulfonyl, S-sulfonamido, N-sulfonamido, N-trihalomethanesulfonamido, carbonyl, aldehyde, trihalomethyl-carbonyl, C-carboxy, O-carboxy, cyano, nitro, halo, cyanato, isocyanato, thiocyanato, isothiocyanato, O-carbamyl, N-carbamyl, O-thiocarbamyl, N-thiocarbamyl, phosphonyl, guanyl, guanidinyl, ureidyl, C-amido, N-amido, amino, quaternary ammonium, -NR¹⁸R¹⁹;
R¹⁸ and R¹⁹ are independently selected from the group consisting of hydrogen, C1-C10 alkyl, cycloalkyl, aryl, carbonyl, acetyl, trihalomethylcarbonyl, C-carboxy, trihalomethanesulfonyl, sulfonyl, C-peptidyl and, combined, a five-member or a six-member heteroalicylic ring;
Z is oxygen;
Q is 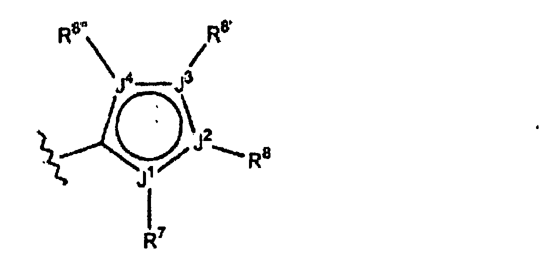 J¹ is selected from the group consisting of oxygen, nitrogen and sulfur;
J², J³ and J⁴ are independently selected from the group consisting of carbon, nitrogen, oxygen and sulfur such that the five-member heteroaryl ring is one known in the chemical arts, it being further understood that, when J², J³ or J⁴ is nitrogen, oxygen or sulfur, R⁸, R^{8'} or R^{8''} respectively, do not exist; likewise when J¹ is oxygen or sulfur, R⁷ does not exist;
R⁷ is selected from the group consisting of hydrogen, C1-C10 alkyl, cycloalkyl, trihalomethylcarbonyl, aryl, guanyl, carboxyl, sulphonyl, and trihalomethanesulfonyl;
one of R⁸, R^{8'} or R^{8''} is H or an - (alk₁) rM group; wherein (alk₁) is CH₂; r is 1 to 3, inclusive; and M is selected from the group consisting of hydroxy, amino, carboxy, morpholinyl, piperazinyl, tetrazolyl, N-carbamyl, O-carbamyl, S-sulfonamido, N-sulfonamido, sulfonyl, -S(O)₂OH and phosphonyl;
the remaining two of R⁸, R^{8'} and R^{8''} are independently selected from the group consisting of:
hydrogen;
unsubstituted C1-C4 alkyl;
C1-C4 alkyl substituted with one or more groups selected from the group consisting of: halo, hydroxyl, unsubstituted C1-C4 alkoxy, amino, s-sulfonamido, -NR¹⁸R¹⁹ or carboxy;
unsubstituted C1-C4 alkoxy;
C1-C4 alkoxy substituted with one or more halogens;
unsubstituted aryl;
aryl substituted with one or more groups selected from the group consisting of unsubstituted C1-C4 alkyl, unsubstituted C1-C4 alkoxy, halo, trihalomethyl, amino, -NR¹⁸R¹⁹, hydroxy or S-sulfonamido;
unsubstituted heteroaryl;
heteroaryl substituted with one or more groups selected from unsubstituted C1-C4 alkyl, unsubstituted C1-C4 alkoxy, halo, trihalomethyl, amino, -NR¹⁸R¹⁹, hydroxy or S-sulfonamido;
unsubstituted heteroalicyclic;
heteroalicyclic substituted with one or more groups selected from unsubstituted C1-C4 alkyl, halo, hydroxy, unsubstituted alkoxy, amino or -NR¹⁸R¹⁹;
halo;
cyano;
carboxy; and
a six member cycloalkyl, aryl, heteroaryl or heteroalicyclic ring formed by combining R⁸ and R^{8'} or R^{8'} and R^{8"};
and physiologically acceptable salts thereof;

As used herein, the term "alkyl" refers to a saturated aliphatic hydrocarbon including straight chain and branched chain groups. The alkyl group has 1 to 10 carbon atoms. Most preferably, it is a lower alkyl having 1 to 4 carbon atoms. The alkyl group may be substituted or unsubstituted. When substituted, the substituent group(s) is one or more individually selected from unsubstituted cycloalkyl selected from the group consisting of cyclopropane, cyclobutane, cyclopentane, cyclopentene, cyclohexane, adamantane, cyclohexadiene, cycloheptane and cycloheptatriene; unsubstituted aryl selected from the group consisting of phenyl, naphthalenyl and anthracenyl; unsubstituted heteroaryl selected from the group consisting of pyrrole, furan, thiophene, imidazole, oxazole, thiazole, pyrazole, pyridine, pyrimidine, quinoline, isoquinoline, purine and carbazole; unsubstituted heteroalicyclic selected from the group consisting of piperidine, morpholine, piperazine, pyrroline, pyrrolidine, dihydrothiophene and tetrahydrofuran; hydroxy; C1-C4 alkoxy, aryloxy; thiohydroxy; C1-C4 thioalkoxy; thioaryloxy; cyano; halo; carbonyl; thiocarbonyl; O-carbamyl; N-carbamyl; O-thiocarbamyl; N-thiocarbamyl; C-amido; N-amido; C-carboxy; O-carboxy; cyanato; isocyanato; thiocyanato; isothiocyanato; nitro; silyl; amino and -NR¹⁸R¹⁹, where R¹⁸ and R¹⁹ are independently selected from the group consisting of hydrogen, unsubstituted C1-C4 alkyl, unsubstituted cycloalkyl as defined above, unsubstituted aryl as defined above, carbonyl, trihalomethylcarbonyl, C-carboxy, trihalomethylsulfonyl, sulfonyl and, combined, a five-member or six-member heteroalicyclic ring as defined above.

A "cycloalkyl" group refers to an all-carbon monocyclic or fused ring (i.e., rings which share an adjacent pair of carbon atoms) wherein one of more of the rings does not have a completely conjugated pi-electron system. The cycloalkyl group is selected from cyclopropane, cyclobutane, cyclopentane, cyclopentene, cyclohexane, adamantane, cyclohexadiene, cycloheptane and cycloheptatriene. A cycloalkyl group may be substituted or unsubstituted. When substituted, the substituent group(s) is one or more individually selected from unsubstituted C1-C4 alkyl, unsubstituted aryl selected from the group consisting of phenyl, naphthalenyl and anthracenyl; unsubstituted heteroaryl selected from the group consisting of pyrrole, furan, thiophene, imidazole, oxazole, thiazole, pyrazole, pyridine, pyrimidine, quinoline, isoquinoline, purine and carbazole; unsubstituted heteroalicyclic selected from the group consisting of piperidine, morpholine, piperazine, pyrroline, pyrrolidine, dihydrothiophene and tetrahydrofuran; hydroxy; C1-C4 alkoxy, aryloxy; thiohydroxy; C1-C4 thioalkoxy; thioaryloxy; cyano; halo; carbonyl; thiocarbonyl; carboxy; O-carbamyl; N-carbamyl; C-amido; N-amido; nitro; amino and -NR¹⁸R¹⁹, with R¹⁸ and R¹⁹ being as defined above.

An "alkenyl" group refers to an alkyl group, as defined herein, consisting of at least two carbon atoms and at least one carbon-carbon double bond.

An "alkynyl" group refers to an alkyl group, as defined herein, consisting of at least two carbon atoms and at least one carbon-carbon triple bond.

An "aryl" group refers to an all-carbon monocyclic or fused-ring polycyclic (i.e. rings which share adjacent pairs of carbon atoms) groups having a completely conjugated pi-electron system. The aryl group is selected from phenyl, naphthalenyl and anthracenyl. The aryl group may be substituted or unsubstituted. When substituted, the substituent group(s) is one or more individually selected from halo; trihalomethyl; unsubstituted C1-C4 alkyl; unsubstituted aryl selected from the group consisting of phenyl, naphthalenyl and anthracenyl; unsubstituted heteroaryl selected from the group consisting of pyrrole, furan, thiophene, imidazole, oxazole, thiazole, pyrazole, pyridine, pyrimidine, quinoline, isoquinoline, purine and carbazole; unsubstituted heteroalicyclic selected from the group consisting of piperidine, morpholine, piperazine, pyrroline, pyrrolidine, dihydrothiophene and tetrahydrofuran; hydroxy; C1-C4 alkoxy, aryloxy; thiohydroxy; C1-C4 thioalkoxy; thioaryloxy; cyano; nitro; carbonyl; thiocarbonyl; C-carboxy; O-carboxy; O-carbamyl; N-carbamyl; O-thiocarbamyl; N-thiocarbamyl; C-amido; N-amido; sulfinyl; sulfonyl; S-sulfonamido; N-sulfonamido; trihalomethanesulfonamido; amino and -NR¹⁸R¹⁹, R¹⁸ and R¹⁹ being defined above.

As used herein, a "heteroaryl" group refers to a monocyclic or fused ring (i.e. rings which share an adjacent pair of atoms) group having in the ring(s) one or more atoms selected from the group consisting of nitrogen, oxygen and sulfur and, in addition, having a completely conjugated pi-electron system. The heteroaryl group is selected from pyrrole, furan, thiophene, imidazole, oxazole, thiazole, pyrazole, pyridine, pyrimidine, quinoline, isoquinoline, purine and carbazole. The heteroaryl group may be substituted or unsubstituted. When substituted, the substituted group (s) is one or more selected from unsubstituted C1-C4 alkyl; unsubstituted cycloalkyl selected from the group consisting of cyclopropane, cyclobutane, cyclopentane, cyclopentene, cyclohexane, adamantane, cyclohexadiene, cycloheptane and cycloheptatriene; unsubstituted aryl selected from the group consisting of phenyl, naphthalenyl and anthracenyl; halo; trihalomethyl; hydroxy; C1-C4 alkoxy, aryloxy; thiohydroxy; C1-C4 thioalkoxy; thioaryloxy; cyano; nitro; carbonyl; thiocarbonyl; sulfonamido; carboxy; sulfinyl; sulfonyl; O-carbamyl; N-carbamyl; O-thiocarbamyl; N-thiocarbamyl; C-amido; N-amido; amino and -NR¹⁸R¹⁹, with R¹⁸ and R¹⁹ being as defined above.

A "heteroalicyclic" group refers to a monocyclic or fused ring group having in the ring(s) one or more atoms selected from the group consisting of nitrogen, oxygen and sulfur. The rings may also have one or more double bonds. However, the rings do not have a completely conjugated pi-electron system. The heteroalicyclic group is selected from piperidine, morpholine, piperazine, pyrroline, pyrrolidine, dihydrothiophene and tetrahydrofuran. The heteroalicyclic ring may be substituted or unsubstituted. When substituted, the substituted group(s) is one or more selected from unsubstituted C1-C4 alkyl; unsubstituted cycloalkyl selected from the group consisting of cyclopropane, cyclobutane, cyclopentane, cyclopentene, cyclohexane, adamantane, cyclohexadiene, cycloheptane and cycloheptatriene; halo; trihalomethyl; hydroxy; C1-C4 alkoxy, aryloxy; thiohydroxy; C1-C4 thioalkoxy; thioaryloxy; cyano; nitro; carbonyl; thiocarbonyl; carboxy; O-carbamyl; N-carbamyl; O-thiocarbamyl; N-thiocarbamyl; sulfinyl; sulfonyl; C-amido; N-amido; amino and -NR¹⁸R¹⁹, with R¹⁸ and R¹⁹ as defined above.

A "hydroxy" group refers to an -OH group.

An "alkoxy" group refers to both an -O-alkyl and an -O-cycloalkyl group, where alkyl and cycloalkyl are as defined above.

An "aryloxy" group refers to both an -O-aryl and an -O-heteroaryl group, where aryl and heteroaryl are as defined above.

A "heteroalicycloxy" group refers to an -0-heteroalicyclic group, where heteroalicyclic is as defined above.

A "thiohydroxy" group refers to an -SH group.

A "thioalkoxy" group refers to both an S-alkyl and an -S-cycloalkyl group, where alkyl and cycloalkyl are as defined above.

A "thioaryloxy" group refers to both an -S-aryl and an -S-heteroaryl group, where aryl and heteroaryl are as defined above.

A "carbonyl" group refers to a -C(=O)-R" group, where R" is selected from the group consisting of hydrogen, alkyl, cycloalkyl, aryl, heteroaryl (bonded through a ring carbon) and heteroalicyclic (bonded through a ring carbon), as defined herein.

A "trihalomethylcarbonyl" group refers to a X₃CC(=O)-group wherein X is a halo group as defined herein.

An "aldehyde" group refers to a carbonyl group where R" is hydrogen.

A "thiocarbonyl" group refers to a -C(=S)-R" group, with R" as defined herein.

An "O-carboxy" group refers to a R"C(=O)O- group, with R" as defined herein.

A "C-carboxy" group refers to a -C(=O)OR" groups with R" as defined herein.

An "acetyl" group refers to a -C(=0)CH₃ group.

A "carboxyalkyl" group refers to - (CH₂)₃C(O)OR" wherein s is 1-6 and R" is as defined above.

A "carboxylic acid" group refers to a C-carboxy group in which R" is hydrogen.

A "halo" group refers to fluorine, chlorine, bromine or iodine.

A "trihalomethyl" group refers to a -CX₃ group wherein X is a halo group as defined herein.

A "trihalomethanesulfonyl" group refers to a X₃CS(=O)₂-groups with X as defined above.

A "cyano" group refers to a -C≡N group.

A "cyanato" group refers to a -CNO group.

An "isocyanato" group refers to a -NCO group.

A "thiocyanato" group refers to a -CNS group.

An "isothiocyanato" group refers to a -NCS group.

A "sulfinyl" group refers to a -S(=O)-R" group, with R" as defined herein.

A "sulfonyl" group refers to a -S(=O)₂R" group, with R" as defined herein.

A "sulfonamido" group refers to a -S(=O)₂ -NR¹⁸R¹⁹, with R¹⁸ and R¹⁹ as defined herein.

A "trihalomethanesulfonamido" group refers to a X₃CS (=O)₂N(R¹⁸) - group with X and R¹⁸ as defined herein.

An "O-carbamyl" group refers to a -OC(=O)NR¹⁸R¹⁹ group with R¹⁸ and R¹⁹ as defined herein.

An "N-carbamyl" group refers to a R¹⁹OC (=O) NR¹⁸- group, with R¹⁸ and R¹⁹ as defined herein.

An "O-thiocarbamyl" group refers to a -OC(=S)-NR¹⁸R¹⁹ group with R¹⁸ and R¹⁹ as defined herein.

An "N-thiocarbamyl" group refers to an R¹⁹OC (=S) NR¹⁸-group, with R¹⁸ and R¹⁹ as defined herein.

An "amino" group refers to an -NR¹⁸R¹⁹ group, with R¹⁸ and R¹⁹ both being hydrogen.

A "quaternary ammonium" group refers to a -⁺NR¹⁸R^{18'}R¹⁹ with R¹⁸ and R¹⁹ as defined herein and R^{18'} defined the same as R¹⁸ and R¹⁹.

A "C-amido" group refers to a -C (=O) -NR¹⁸R¹⁹ group with R¹⁸ and R¹⁹ as defined herein.

An "N-amido" group refers to an R¹⁸C (=O) NR¹⁹- group, with R¹⁸ and R¹⁹ as defined herein.

A "C-thioamido" group refers to a -C(=S)NR¹⁸R¹⁹ group, with R¹⁸ and R¹⁹ as defined herein.

A "nitro" group refers to a -NO₂ group.

A "methylenedioxy" group refers to a -OCH₂O- group wherein the oxygen atoms are bonded to adjacent ring carbon atoms.

An "ethylenedioxy" group refers to a -OCH₂CH₂O- group wherein the oxygen atoms are bonded to adjacent ring carbon atoms.

A "guanyl" group refers to an R¹⁸R¹⁹NC(=N)- group with R¹⁸ and R¹⁹ as defined herein.

A "guanadinyl" group refers to an -R¹⁸NC(=N)NR^{18'}R¹⁹ group with R¹⁸ and R¹⁹ as defined herein, R^{18'} being defined the same as R¹⁸ and R^{19.}

A "ureidyl" group refers to an -NR¹⁸C (=O) NR^{18'} R¹⁹ group with R¹⁸ and R¹⁹ as defined herein and R^{18'} defined the same as R¹⁸ and R¹⁹.

A "phosphonyl" group refers to an -OP(=O)₂OR" group with R" as defined herein.

A "C-peptidyl" group refers to a group formed by the reaction of the 2-amino group of an amino acid with the carboxy group of another amino acid; i.e., a peptidyl group has the general structure -(NCHRC(=O)ₙ- where the R group can be the same or different depending on which amino acid is used to make the peptidyl group, the "C-peptidyl" designation referring to the situation where the peptidyl group is bonded to a non-amino acid molecule through the C(=O) carbon.

A "polar group" refers to a group wherein the nuclei of the atoms covalently bound to each other to form the group do not share the electrons of the covalent bond(s) joining them equally; that is the electron cloud is denser about one atom than another. This results in one end of the covalent bond(s) being relatively negative and the other end relatively positive; i.e., there is a negative pole and a positive pole. Examples of polar groups include, without limitation, hydroxy, alkoxy, carboxy, nitro, cyano, amino, quaternary ammonium, amido, ureidyl, sulfonamido, sulfinyl, sulfonyl, phosphono, morpholino, piperazinyl and tetrazolyl.

A "spirocycloalkyl" group refers to cycloalkyl group which shares a ring carbon atom with another ring.

A "spiroheteroalicyclic" group refers to a heteroalicyclic group which shares a ring carbon atom with another ring.

An "N-hydroxylamino" group refers to a R"ONR¹⁸- group with R" and R¹⁸ as defined herein.

A "polyhydroxyalkyl" group refers to a 1 to 8 carbon, preferably a 1 to 4 carbon unbranched alkyl group substituted with 2 or more, preferably 3 or more hydroxyl groups only.

A "morpholinyl" group refers to a group having the chemical structure:

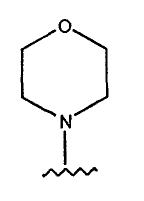

A "tetrazolyl" group refers to a group having the chemical structure:

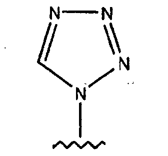

An "aminotriazoly" group refers to a group having the chemical structure:

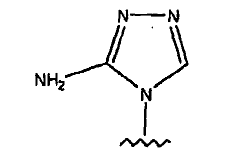

A "1-piperazinyl" group refers to a group having the chemical structure:

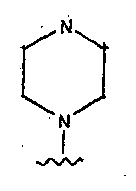

Non-limiting examples of 5-member heteroaryl rings Q known in the chemical arts include the following:

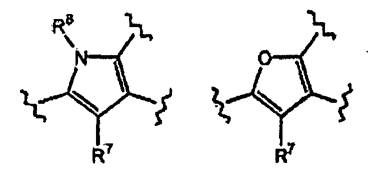

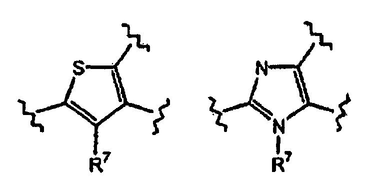

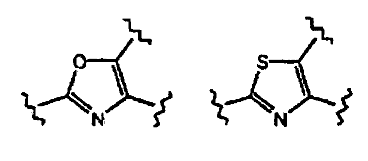

### B. Preferred Structural Features

In a preferred feature of this invention R¹, R² and R⁷ are hydrogen.

In another preferred feature of the invention R³, R⁴, R⁵ and R⁶ are independently selected from the group consisting of:
hydrogen;
unsubstituted C1-C4 alkyl;
C1-C4 alkyl substituted with a group selected from the group consisting of unsubstituted cycloalkyl, halo, hydroxy, unsubstituted C1-C4 alkoxy, C-carboxy, unsubstituted aryl, unsubstituted heteroaryl, unsubstituted heteroalicyclic, amino, quaternary ammonium or -NR¹⁸NR¹⁹;
unsubstituted cycloalkyl;
hydroxy;
unsubstituted C1-C4 alkoxy;
C1-C4 alkoxy substituted with a group selected from the group consisting of one or more halo groups, unsubstituted aryl or unsubstituted heteroaryl;
trihalomethyl;
halo;
unsubstituted aryl;
aryl substituted with one or more groups independently selected from the group consisting of unsubstituted C1-C4 alkyl, C1-C4 alkyl substituted with one or more halo groups, trihalomethyl, halo, hydroxy, or unsubstituted C1-C4 alkoxy;
unsubstituted aryloxy;
aryloxy substituted with one or more groups independently selected from the group consisting of halo, unsubstituted C1-C4 alkyl, C1-C4 alkyl substituted with one or more halo groups, unsubstituted aryl, hydroxy, unsubstituted C1-C4 alkoxy, amino or -NR¹⁸NR¹⁹;
S-sulfonamido;
unsubstituted heteroaryl;
heteroaryl substituted with one or more groups independently selected from the group consisting of hydrogen, unsubstituted C1-C4 alkyl, C1-C4 alkyl sustituted with one or more halo groups, trihalomethyl, halo, hydroxy, unsubstituted C1-C4 alkoxy, unsubstituted aryloxy, amino, S-sulfonamido or -NR¹⁸R¹⁹;
unsubstituted heteroalicyclic;
heteroalicyclic substituted with one or more groups independently selected from the group consisting of unsubstituted C1-C4 alkyl, C1-C4 alkyl substituted with one or more halo groups, unsubstituted C1-C4 alkyl alkoxy, hydroxy, amino, S-sulfonamido or -NR¹⁸R¹⁹;
unsubstituted C1-C4 alkyl C-carboxy;
carboxylic acid;
unsubstituted C1-C4 alkyl carbonyl;
aldehyde;
unsubstituted aryl carbonyl;
acetyl;
S-sulfonamido;
N-sulfonamido;
amino; and,
-NR¹⁸NR¹⁹.

Additional preferred features of this invention are those in which:
Any two of R⁸, R^{8'} or R^{8"} are - (alk₁)rM groups.
J¹ is selected from the group consisting of nitrogen, oxygen and sulphur and J², J³ and J⁴ are carbon.
J¹ and J³ are nitrogen and J² and J⁴ are carbon.

Examples of specific compounds of this invention are shown in Table 1 below.

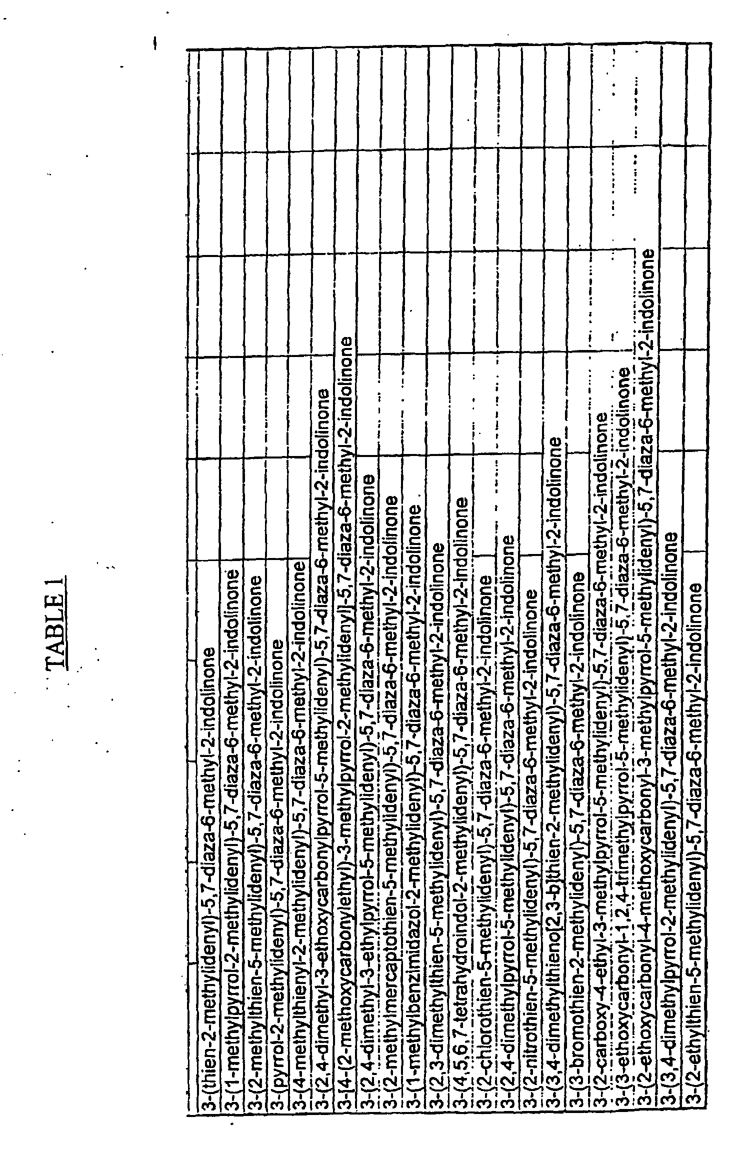

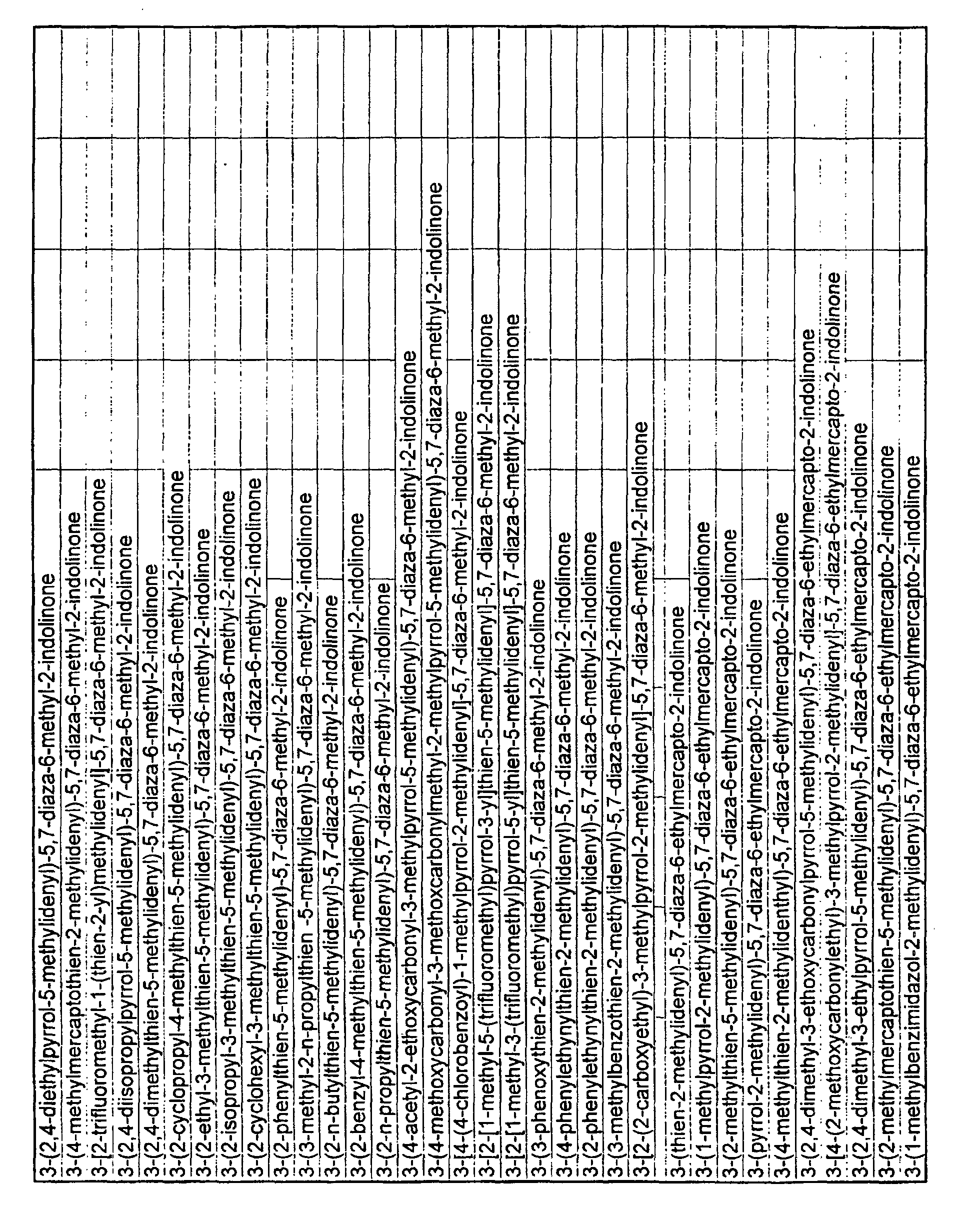

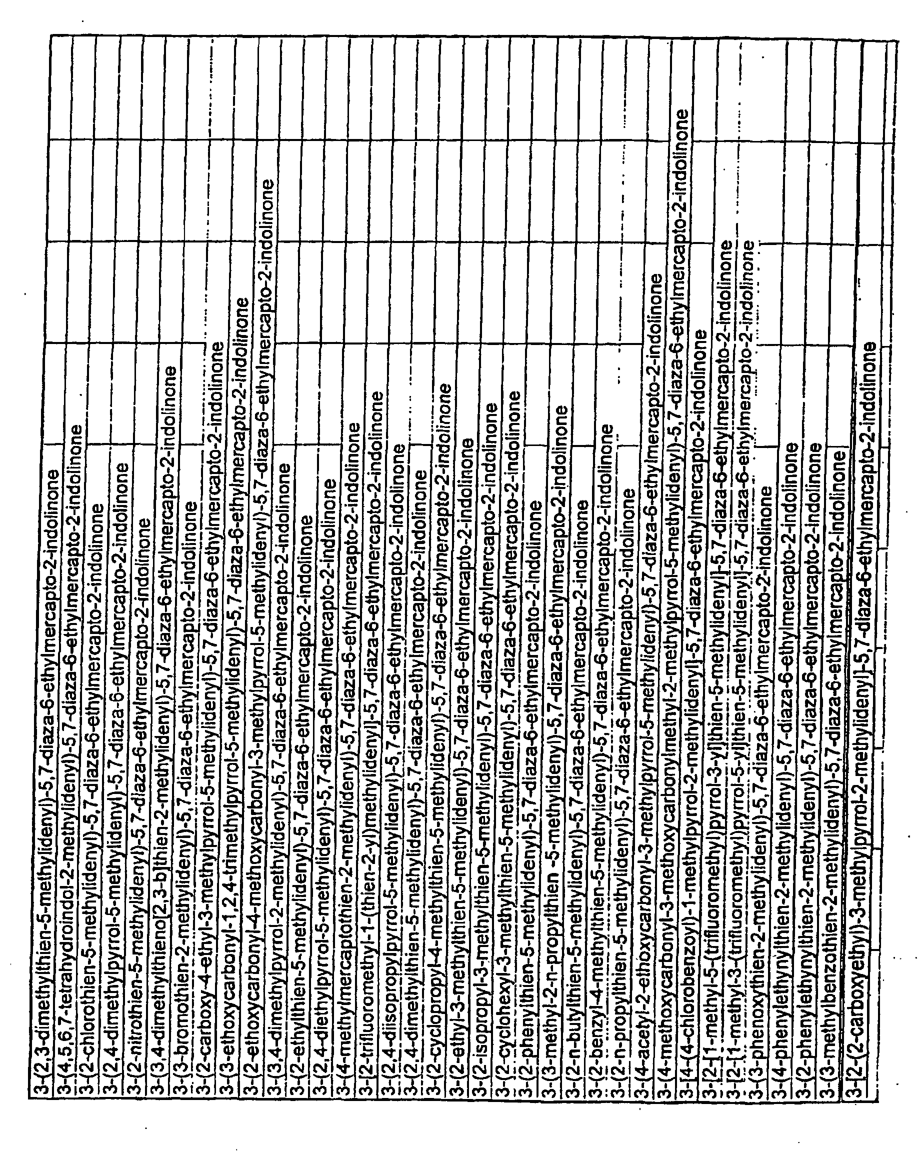

### 2. The Biochemistry

RTK mediated signal transduction is initiated by extracellular interaction with a specific growth factor (ligand), followed by receptor dimerization, transient stimulation of the intrinsic protein tyrosine kinase activity and phosphorylation. Binding sites are thereby created for intracellular signal transduction molecules and lead to the formation of complexes with a spectrum of cytoplasmic signaling molecules that facilitate the appropriate cellular response (e.g., cell division, metabolic effects to the extracellular micro-environment). See, Schlessinger and Ullrich, 1992, Neuron **9**:303-391.

It has been shown that tyrosine phosphorylation sites in growth factor receptors function as high-affinity binding sites for SH2 (src homology) domains of signaling molecules. Fantl et al., 1992, Cell 69:413-423; Songyang *et al*., 1994, Mol. Cell. Biol. 14:2777-2785); Songyang et al., 1993, Cell 72:767-778; and Koch *et al.,* 1991, Science 252:668-678. Several intracellular substrate proteins that associate with RTKs have been identified. They may be divided into two principal groups: (1) substrates which have a catalytic domain; and (2) substrates which lack such domain but serve as adapters and associate with catalytically active molecules. Songyang et al., 1993, Cell 72:767-778. The specificity of the interactions between receptors and SH2 domains of their substrates is determined by the amino acid residues immediately surrounding the phosphorylated tyrosine residue. Differences in the binding affinities between SH2 domains and the amino acid sequences surrounding the phosphotyrosine residues on particular receptors are consistent with the observed differences in their substrate phosphorylation profiles. Songyang et al., 1993, Cell 72:767-778. These observations suggest that the function of each RTK is determined not only by its pattern of expression and ligand availability but also by the array of downstream signal transduction pathways that are activated by a particular receptor. Thus, phosphorylation provides an important regulatory step which determines the selectivity of signaling pathways recruited by specific growth factor receptors, as well as differentiation factor receptors.

STKs, being primarily cytosolic, affect the internal biochemistry of the cell often as a down-line response to a PTK event. STKs have been implicated in the signaling process which initiates DNA synthesis and subsequent mitosis leading to cell proliferation.

Thus, PK signal transduction results in, among other responses, cell proliferation, differentiation, growth and metabolism. Abnormal cell proliferation may result in a wide array of disorders and diseases, including the development of neoplasia such as carcinoma, sarcoma, leukemia, glioblastoma, hemangioma, psoriasis, arteriosclerosis, arthritis and diabetic retinopathy (or other disorders related to uncontrolled angiogenesis and/or vasculogenesis).

A precise understanding of the mechanism by which the compounds of this invention inhibit PKs is not required in order to practice the present invention. However, while not hereby being bound to any particular mechanism or theory, it is believed that the compounds interact with the amino acids of the catalytic region of PKs. PKs typically possess a bi-lobate structure wherein ATP appears to bind in the cleft between the two lobes in a region where the amino acids are conserved among PKs. Inhibitors of PKs are believed to bind by non-covalent interactions such as hydrogen bonding, van der Waals forces and ionic interactions in the same general region where the aforesaid ATP binds to the PKs. More specifically, it is thought that the 2-indolinone component of the compounds of this invention binds in the general space normally occupied by the adenine ring of ATP. Specificity of a particular molecule for a particular PK could arise as the result of additional interactions between the various substituents on the 2-indolinone core with amino acid domains specific to particular PKs. Thus, different indolinone substituents may contribute to preferential binding to particular PKs. The ability to select those compounds active at different ATP (or other nucleotide) binding sites makes the compounds useful for targeting any protein with such a site; i.e., not only PKs but protein phosphatases as well. The compounds disclosed herein thus have utility for in vitro assays on such proteins and for in vivo therapeutic effects through such proteins.

In another aspect, this invention relates to the use of a compound according to the invention in the manufacture of a medicament for the treatment of a disease selected from a cancer selected from the group consisting of squamous cell carcinoma, astrocytoma, glioblastoma, breast cancer, glioma, melanoma, lung cancer, Small-cell lung cancer, bladder cancer, ovarian cancer, prostate cancer, and head and neck cancer; a proliferation disorder selected from the group consisting of restinosis, fibrosis, psoriasis, osteoarthritis and rheumatoid arthritis; diabetes, autoimmune disease, an inflammatory disorder and angiogenesis.

### 3. Pharmacological Compositions and Therapeutic Apiplications

In a further aspect the present invention relates to a pharmaceutical composition comprising a compound or salt as described herein and a pharmaceutically acceptable carrier or diluent.

### A. Routes of Administration.

As used herein, "administer" or "administration" refers to the delivery of a compound, salt or prodrug of the present invention or of a pharamacological composition containing a compound, salt or prodrug of this invention to an organism for the purpose of prevention or treatment of a PK-related disorder.

Suitable routes of administration may include, without limitation, oral, rectal, transmucosal or intestinal administration; or, intramuscular, subcutaneous, intramedullary, intrathecal, direct intraventricular, intravenous, intraperitoneal, intranasal, or intraocular injections.

Alternately, one may administer the compound in a local rather than systemic manner, for example, via injection of the compound directly into a solid tumor, often in a depot or sustained release formulation.

Furthermore, one may administer the drug in a targeted drug delivery system, for example, in a liposome coated with tumor-specific antibody. The liposomes will be targeted to and taken up selectively by the tumor.

### Composition/Formulation

Pharmacological compositions of the present invention may be manufactured by processes well known in the art; e.g., by means of conventional mixing, dissolving, granulating, dragee-making, levigating, emulsifying, encapsulating, entrapping or lyophilizing processes.

Pharmacological compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

For injection, the compounds of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmacological preparations for oral use can be made with the use of a solid excipient, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmacological compositions which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichloro-tetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmacological compositions for parenteral administration include aqueous solutions of the active compounds in water soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The pharmacological compositions herein also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Many of the PK modulating compounds of the invention may be provided as physiologically acceptable salts wherein the claimed compound may form the negatively or the positively charged species. Examples of salts in which the compound forms the positively charged moiety include, without limitation, quaternary ammonium (defined elsewhere herein), salts such as the hydrochloride, sulfate, carbonate, lactate, tartrate, maleate, succinate, etc. formed by the reaction of an amino group with the appropriate acid. Salts in which the compound forms the negatively charged species include, without limitation, the sodium, potassium, calcium and magnesium salts formed by the reaction of a carboxylic acid group in the molecule with the appropriate base (e.g. sodium hydroxide (NaOH), potassium hydroxide (KOH), Calcium hydroxide (Ca(OH)₂), etc.).

### C. Dosage.

Pharmacological compositions suitable for use in the present invention include compositions wherein the active ingredients are contained in an amount effective to achieve its intended purpose.

More specifically, a therapeutically effective amount means an amount of compound effective to prevent, alleviate or ameliorate symptoms of disease or prolong the survival of the subject being treated.

Determination of a therapeutically effective amount is well within the capability of those skilled in the art, especially in light of the detailed disclosure provided herein.

For any compound used in the methods of the invention, the therapeutically effective amount or dose can be estimated initially from cell culture assays. Then, the dosage can be formulated for use in animal models so as to achieve a circulating concentration range that includes the IC₅₀ as determined in cell culture (i.e., the concentration of the test compound which achieves a half-maximal inhibition of the PK activity). Such information can then be used to more accurately determine useful doses in humans.

Toxicity and therapeutic efficacy of the compounds described herein can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Compounds which exhibit high therapeutic indices are preferred. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide plasma levels of the active moiety which are sufficient to maintain the kinase modulating effects, or minimal effective concentration (MEC). The MEC will vary for each compound but can be estimated from in vitro data; e.g., the concentration necessary to achieve 50-90% inhibition of the kinase using the assays described herein. Dosages necessary to achieve the MEC will depend on individual characteristics and route of administration. However, HPLC assays or bioassays can be used to determine plasma concentrations.

Dosage intervals can also be determined using MEC value. Compounds should be administered using a regimen which maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered will, of course, be dependent on the subject being treated, on the subject's weight, the severity of the affliction, the manner of administration and the judgment of the prescribing physician.

### D. Packaging.

The compositions may, if desired, be presented in a pack or dispenser device, such as an FDA approved kit, which may contain one or more unit dosage forms containing the active ingredient. The pack may for example comprise metal or plastic foil, such as a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or human or veterinary administration. Such notice, for example, may be of the labeling approved by the U.S. Foods and Drug Administration for prescription drugs or of an approved product insert. Compositions comprising a compound of the invention formulated in a compatible pharmaceutical carrier may also be prepared, placed in an appropriate container, and labeled for treatment of an indicated condition. Suitable conditions indicated on the label may include treatment of a tumor, inhibition of angiogenesis, treatment of fibrosis, diabetes, and the like.

### 4. Synthesis

The compounds of this invention, as well as the precursor 2-oxindoles and aldehydes, may be readily synthesized using techniques well known in the chemical arts. It will be appreciated by those skilled in the art that other synthetic pathways for forming the compounds of the invention are available and that the following is offered by way of example and not limitation.

### General synthetic procedure.

The following general methodology may be employed to prepare the compounds of this invention:

The appropriately substituted 2-oxindole (1 equiv.), the appropriately substituted aldehyde (1.2 equiv.) and piperidine (0.1 equiv.) are mixed with ethanol (1-2 ml/mmol 2-oxindole) and the mixture is then heated at 90° C for 3 to 5 hours After cooling, the reaction mixture is concentrated and acidified to pH 3. The precipitate which forms is filtered, washed with water to pH 7 and then cold ethanol, ethyl acetate and/or hexane and vacuum dried to yield the target compound. The product may optionally be further purified by chromatography.

### Aldehydes

Aldehydes useful for the synthesis of compounds of this invention can be prepared by numerous synthetic procedures well known to those skilled in the art. For example, the procedures described in the following publications can be employed to give some of the aldehydes of this invention:
J. Med. Chem.,1993, 36(23), 3674-3685;
Chem. Commun., 1966, 393.
Chem. Heterocyl.Compd.(EN), 1974, 10, 50-52; and
J. Chem. Soc. Perkin Trans., 1:EN, 1974, 1237-1243.
Table 2 lists additional aldehydes which can be used to make compounds of this invention.

### 5. BIOLOGICAL EVALUATION

It will be appreciated that, in any given series of compounds, a spectrum of biological activities will be obtained. In its preferred embodiments, this invention relates to novel 2-indolinones demonstrating the ability to modulate RTK, CTK, and STK activity. The following assays are employed to select those compounds demonstrating the optimal degree of the desired activity.

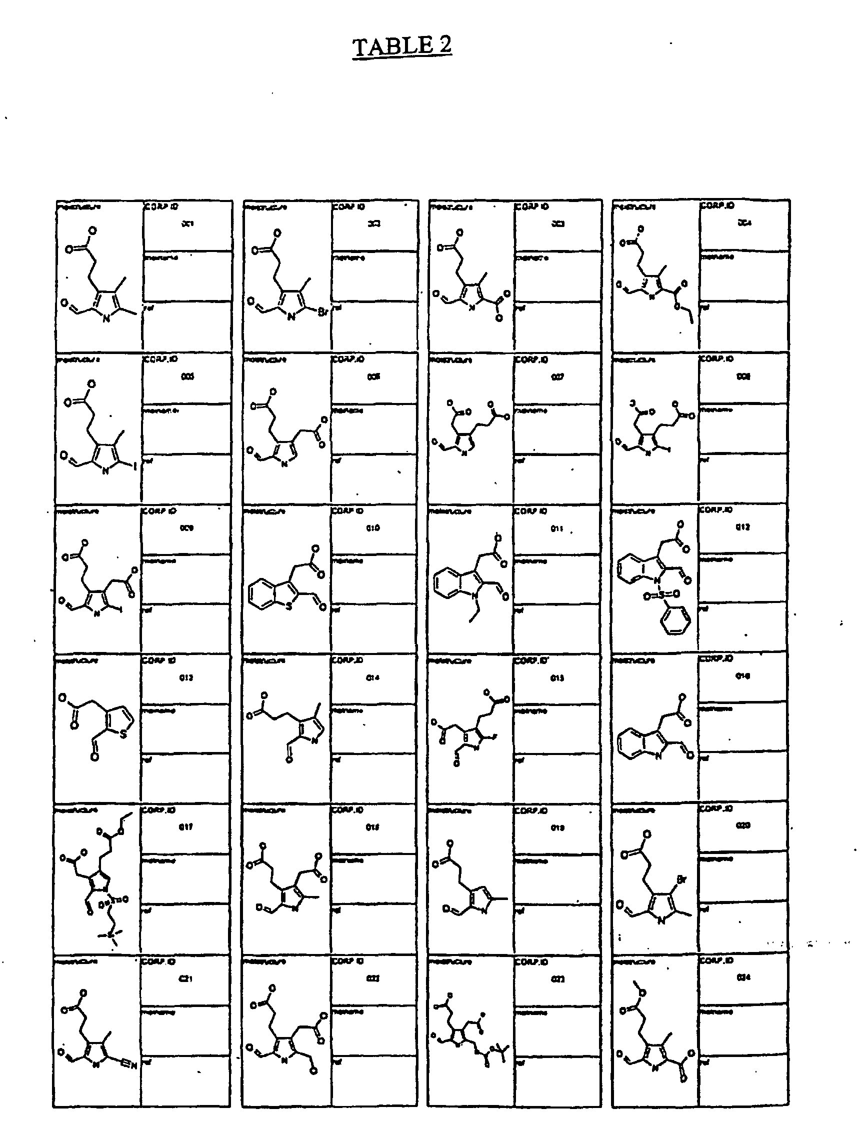

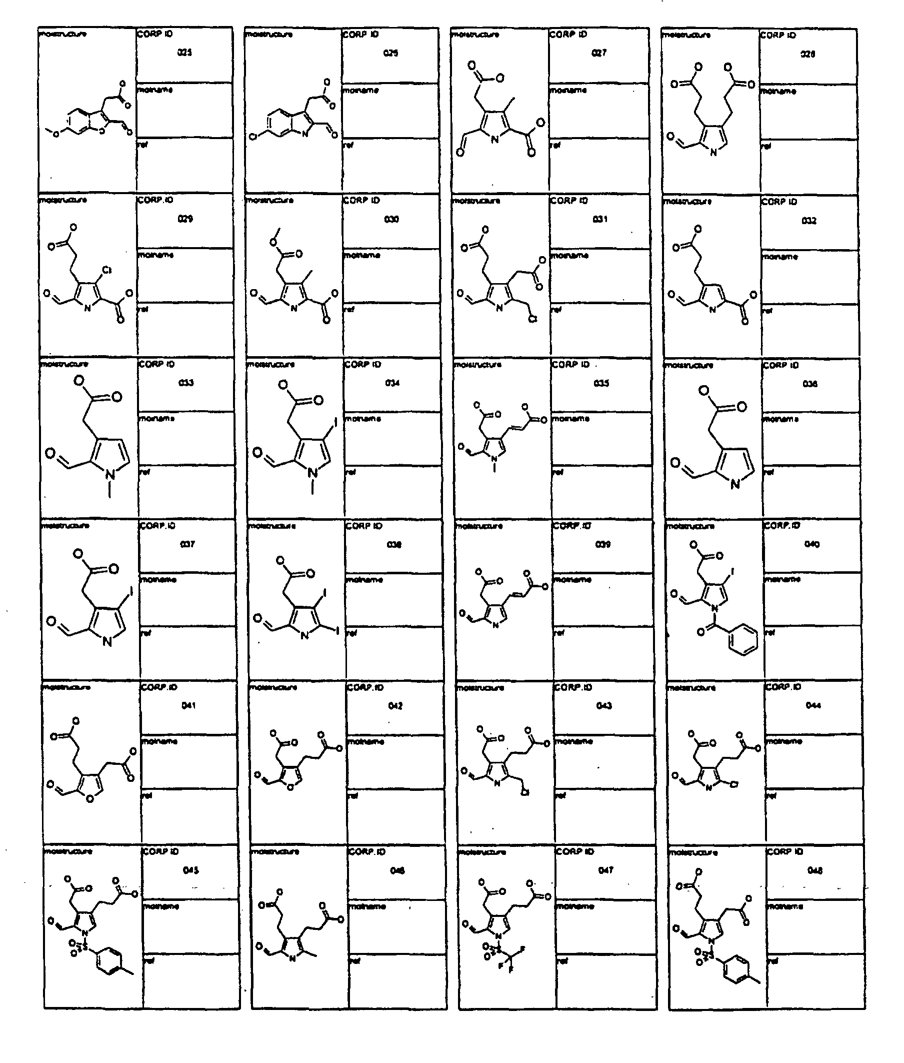

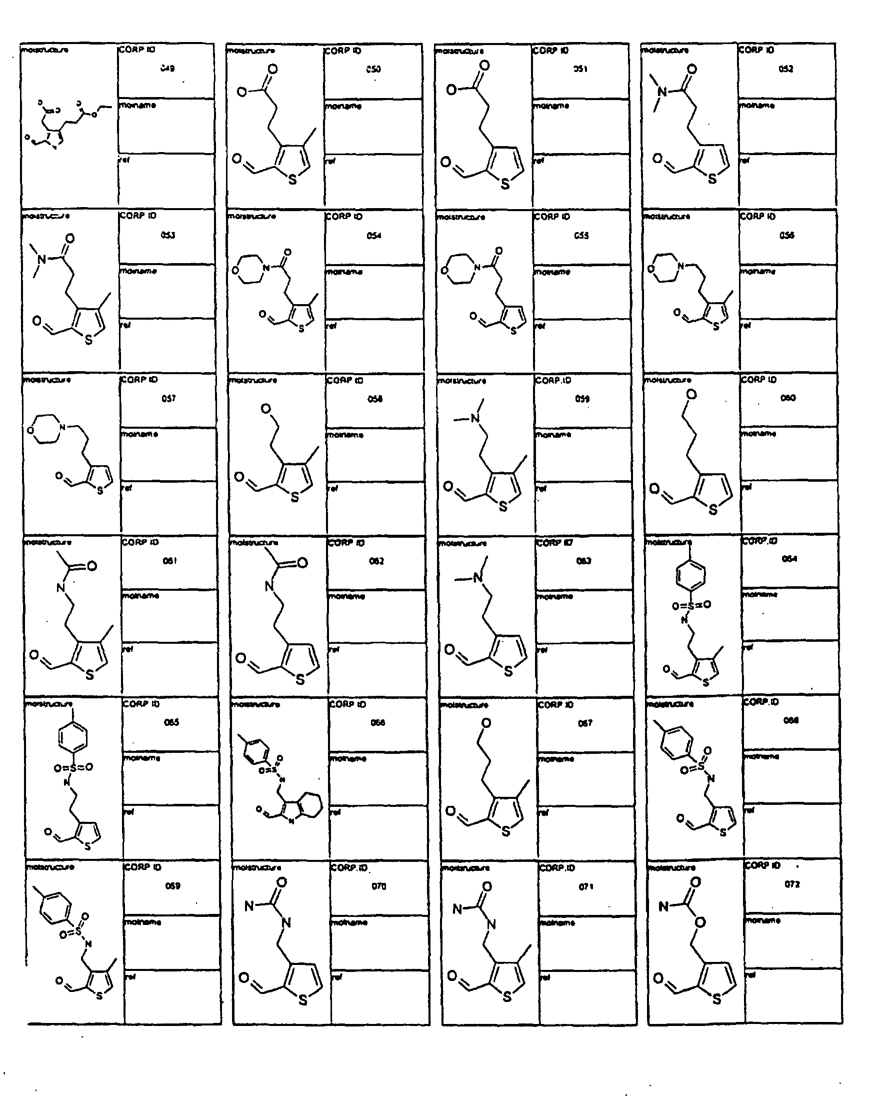

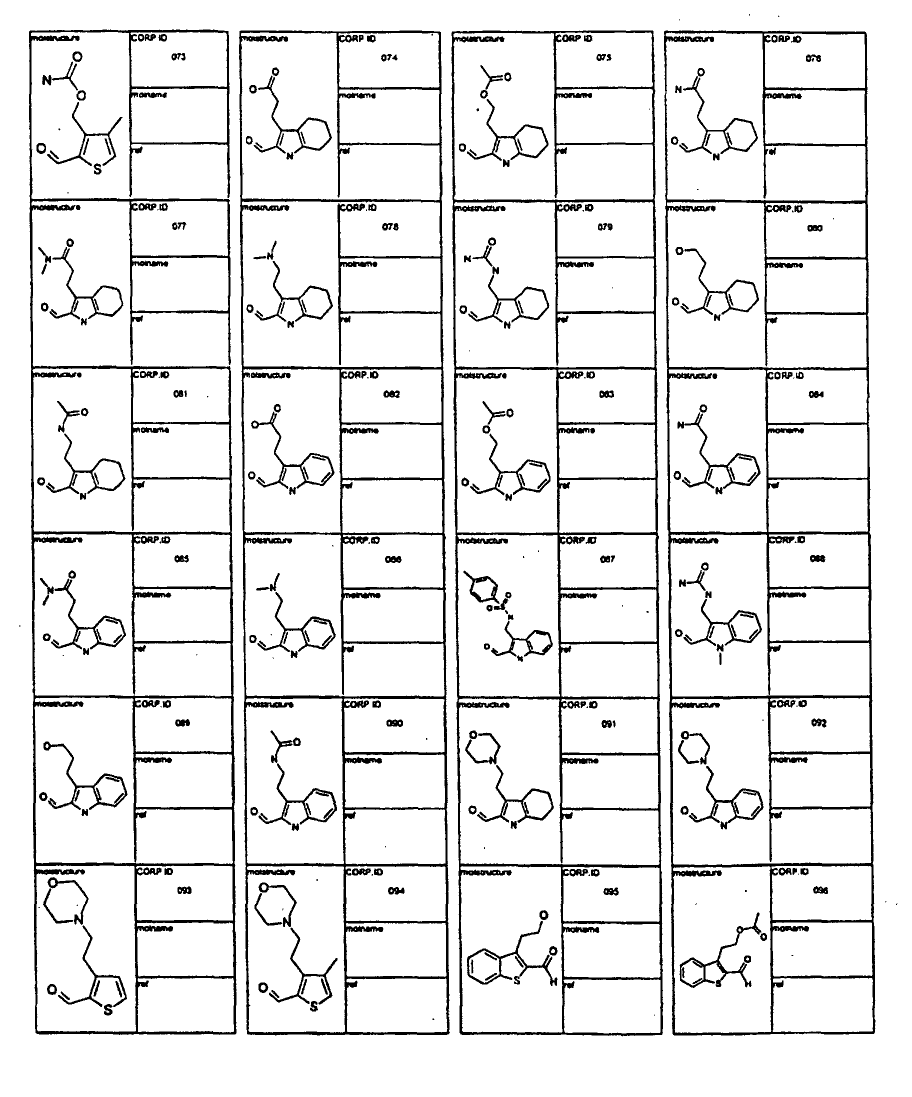

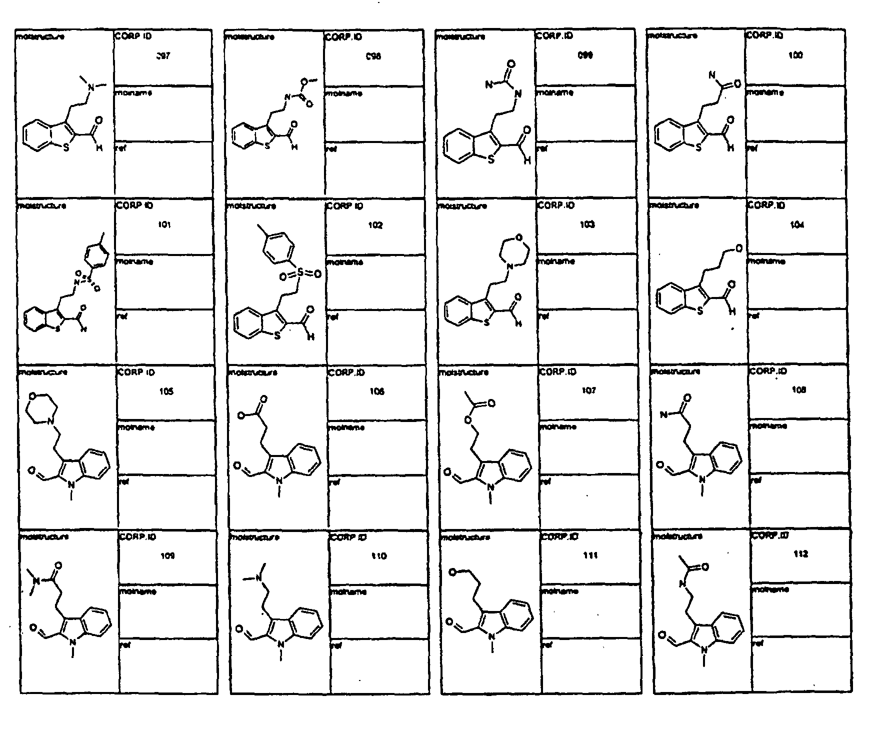

### Assay Procedures.

The following *in vitro* assays may be used to determine the level of activity and effect of the different compounds of the present invention on one or more of the PKs. Similar assays can be designed along the same lines for any PK using techniques well known in the art.

The cellular/catalytic assays described herein are performed in an ELISA format. The general procedure is a follows: a compound is introduced to cells expressing the test kinase, either naturally or recombinantly, for some period of time after which, if the test kinase is a receptor, a ligand known to activate the receptor is added. The cells are lysed and the lysate is transferred to the wells of an ELISA plate previously coated with a specific antibody recognizing the substrate of the enzymatic phosphorylation reaction. Non-substrate components of the cell lysate are washed away and the amount of phosphorylation on the substrate is detected with an antibody specifically recognizing phosphotyrosine compared with control cells that were not contacted with a test compound.

The cellular/biologic assays described herein measure the amount of DNA made in response to activation of a test kinase, which is a general measure of a proliferative response. The general procedure for this assay is as follows: a compound is introduced to cells expressing the test kinase, either naturally or recombinantly, for some period of time after which, if the test kinase is a receptor, a ligand known to activate the receptor is added. After incubation at least overnight, a DNA labeling reagent such as Bromodeoxy-uridine (BrdU) or 3H-thymidine is added. The amount of labeled DNA is detected with either an anti-BrdU antibody or by measuring radioactivity and is compared to control cells not contacted with a test compound.

### 1. Cellular/Catalytic Assays

Enzyme linked immunosorbent assays (ELISA) may be used to detect and measure the presence of PK activity. The ELISA may be conducted according to known protocols which are described in, for example, Voller, et al., 1980, "Enzyme-Linked Immunosorbent Assay," In: Manual of Clinical Immunology, 2d ed., edited by Rose and Friedman, pp 359-371 Am. Soc. Of Microbiology, Washington, D.C.

The disclosed protocol may be adapted for determining activity with respect to a specific PK. For example, the preferred protocols for conducting the ELISA experiments for specific PKs is provided below. Adaptation of these protocols for determining a compound's activity for other members of the RTK family, as well as for CTKs and STKs, is well within the scope of knowledge of those skilled in the art.

### a. FLK-1

An ELISA assay was conducted to measure the kinase activity of the FLK-1 receptor and more specifically, the inhibition or activation of TK activity on the FLK-1 receptor. Specifically, the following assay was conducted to measure kinase activity of the FLK-1 receptor in cells genetically engineered to express Flk-1.

### Materials And Methods.

Materials. The following reagents and supplies were used:
a. Corning 96-well ELISA plates (Corning Catalog No. 25805-96);
b. Cappel goat anti-rabbit IgG (catalog no. 55641);
c. PBS (Gibco Catalog No. 450-1300EB);
d. TBSW Buffer (50 mM Tris (pH 7.2), 150 mM NaCl. and 0.1% Tween-20);
e. Ethanolamine stock (10% ethanolamine (pH 7.0), stored at 4°C);
f. HNTG buffer (20mM HEPES buffer (pH 7.5), 150mM NaCl, 0.2% Triton X-100, and 10% glycerol);
g. EDTA (0.5 M (pH 7.0) as a 100X stock);
h. Sodium orthovanadate (0.5 M as a 100X stock);
i. Sodium pyrophosphate (0.2 M as a 100X stock);
j. NUNC 96 well V bottom polypropylene plates (Applied Scientific Catalog No. AS-72092);
k. NIH3T3 C7#3 Cells (FLK-1 expressing cells);
l. DMEM with 1X high glucose L-Glutamine (catalog No. 11965-050);
m. FBS, Gibco (catalog no. 16000-028);
n. L-glutamine, Gibco (catalog no. 25030-016);
o. VEGF, PeproTech, Inc. (catalog no. 100-20)(kept as 1 µg/100 µl stock in Milli-Q dH₂O and stored at -20°C;
p. Affinity purified anti-FLK-1 antiserum;
q. UB40 monoclonal antibody specific for phosphotyrosine *(see,* Fendley, *et al.,* 1990, *Cancer Research* 50:1550-1558);
r. EIA grade Goat anti-mouse IgG-POD (BioRad catalog no. 172-1011);
s. 2,2-azino-bis(3-ethylbenz-thiazoline-6-sulfonic acid (ABTS) solution (100mM citric acid (anhydrous), 250 mM Na₂HPO₄ (pH 4.0), 0.5 mg/ml ABTS (Sigma catalog no. A-1888)), solution should be stored in dark at 4°C until ready for use;
t. H₂O₂ (30% solution)(Fisher catalog no. H325);
u. ABTS/H₂O₂ (15ml ABTS solution, 2 µl H₂O₂) prepared 5 minutes before use and left at room temperature;
v. 0.2 M HCl stock in H₂O;
w. dimethylsulfoxide (100%)(Sigma Catalog No. D-8418); and
y. Trypsin-EDTA (Gibco BRL Catalog No. 25200-049).

Protocol. The following protocol was used for conducting the assay:
1. Coat Corning 96-well ELISA plates with 1.0µg per well Cappel Anti-rabbit IgG antibody in 0.1M Na₂CO₃ pH 9.6. Bring final volume to 150 µl per well. Coat plates overnight at 4°C. Plates can be kept up to two weeks when stored at 4°C.
2. Grow cells in Growth media(DMEM, supplemented with 2.0mM L-Glutamine, 10% FBS) in suitable culture dishes until confluent at 37°C, 5% CO₂.
3. Harvest cells by trypsinization and seed in Corning 25850 polystyrene 96-well round bottom cell plates, 25.000 cells/well in 200µl of growth media.
4. Grow cells at least one day at 37°C, 5% CO₂.
5. Wash cells with D-PBS 1X.
6. Add 200µl/well of starvation media (DMEM, 2.0mM 1-Glutamine, 0.1% FBS). Incubate overnight at 37°C, 5% CO₂.
7. Dilute Compounds 1:20 in polypropylene 96 well plates using starvation media. Dilute dimethylsulfoxide 1:20 for use in control wells.
8. Remove starvation media from 96 well cell culture plates and add 162 µl of fresh starvation media to each well.
9. Add 18µl of 1:20 diluted Compound dilution (from step 7) to each well plus the 1:20 dimethylsulfoxide dilution to the control wells (+/- VEGF), for a final dilution of 1:200 after cell stimulation. Final dimethylsulfoxide is 0.5%. Incubate the plate at 37°C, 5% CO₂ for two hours.
10. Remove unbound antibody from ELISA plates by inverting plate to remove liquid. Wash 3 times with TBSW + 0.5% ethanolamine, pH 7.0. Pat the plate on a paper towel to remove excess liquid and bubbles.
11. Block plates with TBSW + 0.5% Ethanolamine, pH 7.0, 150 µl per well. Incubate plate thirty minutes while shaking on a microtiter plate shaker.
12. Wash plate 3 times as described in step 10.
13. Add 0.5µg/well affinity purified anti-FLU-1 polyclonal rabbit antiserum. Bring final volume to 150µl/well with TBSW + 0.5% ethanolamine pH 7.0. Incubate plate for thirty minutes while shaking.
14. Add 180 µl starvation medium to the cells and stimulate cells with 20µl/well 10.0mM sodium ortho vanadate and 500 ng/ml VEGF (resulting in a final concentration of 1.0mM sodium ortho vanadate and 50ng/ml VEGF per well) for eight minutes at 37°C, 5% CO₂. Negative control wells receive only starvation medium.
15. After eight minutes, media should be removed from the cells and washed one time with 200µl/well PBS.
16. Lyse cells in 150µl/well HNTG while shaking at room temperature for five minutes. HNTG formulation includes sodium ortho vanadate, sodium pyrophosphate and EDTA.
17. Wash ELISA plate three times as described in step 10.
18. Transfer cell lysates from the cell plate to ELISA plate and incubate while shaking for two hours. To transfer cell lysate pipette up and down while scrapping the wells.
19. Wash plate three times as described in step 10.
20. Incubate ELISA plate with 0.02µg/well UB40 in TBSW + 05% ethanolamine. Bring final volume to 150µl/well. Incubate while shaking for 30 minutes.
21. Wash plate three times as described in step 10.
22. Incubate ELISA plate with 1:10,000 diluted EIA grade goat anti-mouse IgG conjugated horseradish peroxidase in TBSW + 0.5% ethanolamine, pH 7.0. Bring final volume to 150µl/well. Incubate while shaking for thirty minutes.
23. Wash plate as described in step 10.
24. Add 100 µl of ABTS/H₂O₂ solution to well. Incubate ten minutes while shaking.
25. Add 100 µl of 0.2 M HCl for 0.1 M HCl final to stop the color development reaction. Shake 1 minute at room temperature. Remove bubbles with slow stream of air and read the ELISA plate in an ELISA plate reader at 410 nm.

### b. HER-2 ELISA

### Assay 1: EGF Receptor-HER2 Chimeric Receptor Assay In Whole Cells.

HER2 kinase activity in whole EGFR-NIH3T3 cells was measured as described below:
Materials and Reagents. The following materials and reagents were used to conduct the assay:
a. EGF: stock concentration: 16.5 ILM; EGF 201, TOYOBO, Co., Ltd. Japan.
b. 05-101 (UBI) (a monoclonal antibody recognizing an EGFR extracellular domain).
Anti-phosphotyrosine antibody (anti-Ptyr) (polyclonal) (*see*, Fendley, *et al., supra).*
d. Detection antibody: Goat anti-rabbit 1gG horse radish peroxidase conjugate, TAGO, Inc., Burlingame, CA.
e. TBST buffer:

| | |
|---|---|
| Tris-HCl, pH 7.2 | 50 mM |
| NaCl | 150 mM |
| Triton X-100 | 0.1 |

f. HNTG 5X stock:

| | |
|---|---|
| HEPES | 0.1 M |
| NaCl | 0.75 M |
| Glycerol | 50% |
| Triton X-100 | 1.0% |

g. ABTS stock:

| | |
|---|---|
| Citric Acid | 100 mM |
| Na₂HPO₄ | 250 mM |
| HCl, conc. | 0.5 pM |
| ABTS* | 0.5mg/ml |
| *(2,2'-azinobis(3-ethylbenzthiazolinesulfonic acid)). | |
| Keep solution in dark at 4°C until use. | |

h. Stock reagents of:

| | |
|---|---|
| EDTA 100 mM pH 7.0 | |
| Na₃VO₄ | 0.5 M |
| Na₄(P₂O₇) | 0.2 M |

Procedure. The following protocol was used:

### A. Pre-coat ELISA Plate

1. Coat ELISA plates (Corning, 96 well, Cat. #25805-96) with 05-101 antibody at 0.5 g per well in PBS, 100 µl final volume/well, and store overnight at 4°C. Coated plates are good for up to 10 days when stored at 4°C.
2. On day of use, remove coating buffer and replace with 100 µl blocking buffer (5% Carnation Instant Non-Fat Dry Milk in PBS). Incubate the plate, shaking, at room temperature (about 23°C to 25°C) for 30 minutes. Just prior to use, remove blocking buffer and wash plate 4 times with TBST buffer.

### B. Seeding Cells

1. An NIH3T3 cell line overexpressing a chimeric receptor containing the EGFR extracellular domain and intracellular HER2 kinase domain can be used for this assay.
2. Choose dishes having 80-90% confluence for the experiment. Trypsinize cells and stop reaction by adding 10% fetal bovine serum. Suspend cells in DMEM medium (10% CS DMEM medium) and centrifuge once at 1500 rpm, at room temperature for 5 minutes.
3. Resuspend cells in seeding medium (DMEM, 0.5% bovine serum), and count the cells using trypan blue. Viability above 90% is acceptable. Seed cells in DMEM medium (0.5% bovine serum) at a density of 10,000 cells per well, 100 µl per well, in a 96 well microtiter plate. Incubate seeded cells in 5% CO₂ at 37°C for about 40 hours.

### C. Assay Procedures

1. Check seeded cells for contamination using an inverted microscope. Dilute drug stock (10 mg/ml in DMSO) 1:10 in DMEM medium, then transfer 5 µl to a TBST well for a final drug dilution of 1:200 and a final DMSO concentration of 1%. Control wells receive DMSO alone. Incubate in 5% CO₂ at 37°C for two hours.
2. Prepare EGF ligand: dilute stock EGF in DMEM so that upon transfer of 10 µl dilute EGF (1:12 dilution), 100 nM final concentration is attained.
3. Prepare fresh HNTG* sufficient for 100 µl per well; and place on ice.

| | |
|---|---|
| HNTG* (10 ml): | |
| HNTG stock | 2.0 ml |
| milli-Q H₂O | 7.3 ml |
| EDTA, 100 mM, pH 7.0 | 0.5 ml |
| Na₃VO₄, 0.5 M | 0.1 ml |
| Na₄(P₂O₇), 0.2 M | 0.1 ml |

4. After 120 minutes incubation with drug, add prepared SGF ligand to cells, 10 µl per well, to a final concentration of 100 nM. Control wells receive DMEM alone. Incubate, shaking, at room temperature, for 5 minutes.
5. Remove drug, EGF, and DMEM. Wash cells twice with PBS. Transfer HNTG* to cells, 100 µl per well. Place on ice for 5 minutes. Meanwhile, remove blocking buffer from other ELISA plate and wash with TBST as described above.
6. With a pipette tip securely fitted to a micropipettor, scrape cells from plate and homogenize cell material by repeatedly aspirating and dispensing the HNTG* lysis buffer. Transfer lysate to a coated, blocked, and washed ELISA plate. Incubate shaking at room temperature for one hour.
7. Remove lysate and wash 4 times with TBST. Transfer freshly diluted anti-Ptyr antibody to ELISA plate at 100 µl per well. Incubate shaking at room temperature for 30 minutes in the presence of the anti-Ptyr antiserum (1:3000 dilution in TBST).
8. Remove the anti-Ptyr antibody and wash 4 times with TBST. Transfer the freshly diluted TAGO anti-rabbit IgG antibody to the ELISA plate at 100 µl per well. Incubate shaking at room temperature for 30 minutes (anti-rabbit IgG antibody: 1:3000 dilution in TBST).
9. Remove TAGO detection antibody and wash 4 times with TBST. Transfer freshly prepared ABTS/H₂O₂ solution to ELISA plate, 100 µl per well. Incubate shaking at room temperature for 20 minutes. (ABTS/H₂O₂ solution: 1.0 µl 30% H₂O₂ in 10 ml ABTS stock).
10. Stop reaction by adding 50 µl 5N H₂SO₄ (optional), and determine O.D. at 410 nm.
11. The maximal phosphotyrosine signal is determined by subtracting the value of the negative controls from the positive controls. The percent inhibition of phosphotyrosine content for extract-containing wells is then calculated, after subtraction of the negative controls.

### c. PDGF-R ELISA

All cell culture media, glutamine, and fetal bovine serum were purchased from Gibco Life Technologies (Grand Island, NY) unless otherwise specified. All cells were grown in a humid atmosphere of 90-95% air and 5-10% CO₂ at 37°C. All cell lines were routinely subcultured twice a week and were negative for mycoplasma as determined by the Mycotect method (Gibco).

For ELISA assays, cells (U1242, obtained from Joseph Schlessinger, NYU) were grown to 80-90% confluency in growth medium (MEM with 10% FBS, NEAA, 1 mM NaPyr and 2 mM GLN) and seeded in 96-well tissue culture plates in 0.5% serum at 25,000 to 30,000 cells per well. After overnight incubation in 0.5% serum-containing medium, cells were changed to serum-free medium and treated with test compound for 2 hr in a 5% CO₂, 37°C incubator. Cells were then stimulated with ligand for 5-10 minute followed by lysis with HNTG (20 mM Hepes, 150 mM NaCl, 10% glycerol, 5 mM EDTA, 5 mM Na₃VO₄, 0.2% Triton X-100, and 2 mM NaPyr). Cell lysates (0.5 mg/well in PBS) were transferred to ELISA plates previously coated with receptor-specific antibody and which had been blocked with 5% milk in TBST (50 mM Tris-HCl pH 7.2, 150 mM NaCl and 0.1% Triton X-100) at room temperature for 30 min. Lysates were incubated with shaking for 1 hour at room temperature. The plates were washed with TBST four times and then incubated with polyclonal anti-phosphotyrosine antibody at room temperature for 30 minutes. Excess anti-phosphotyrosine antibody was removed by rinsing the plate with TBST four times. Goat anti-rabbit IgG antibody was added to the ELISA plate for 30 min at room temperature followed by rinsing with TBST four more times. ABTS (100 mM citric acid, 250 mM Na₂HPO₄ and 0.5 mg/mL 2,2'-azino-bis(3-ethylbenzthiazoline-6-sulfonic acid)) plus H₂O₂ (1.2 mL 30% H₂O₂ to 10 ml ABTS) was added to the ELISA plates to start color development. Absorbance at 410 nm with a reference wavelength of 630 nm was recorded about 15 to 30 min after ABTS addition.

### d. IGF-I RECEPTOR ELISA

The following protocol may be used to measure phosphotyrosine level on IGF-I receptor, which indicates IGF-I receptor tyrosine kinase activity.

Materials And Reagents. The following materials and reagents were used:
a. The cell line used in this assay is 3T3/IGF-1R, a cell line genetically engineered to overexpresses IGF-1 receptor.
b. NIH3T3/IGF-1R is grown in an incubator with 5% CO₂ at 37°C. The growth media is DMEM + 10% FBS (heat inactivated)+ 2mM L-glutamine.
c. Affinity purified anti-IGF-1R antibody 17-69.
d. D-PBS:

| | |
|---|---|
| KH₂PO₄ | 0.20 g/l |
| K₂HPO₄ | 2.16 g/l |
| KCl | 0.20 g/l |
| NaCl | 8.00 g/l (pH 7.2) |

e. Blocking Buffer: TBST plus 5% Milk (Carnation Instant Non-Fat Dry Milk).
f. TBST buffer:

| | |
|---|---|
| Tris-HCl | 50 mM |
| NaCl | 150mM (pH 7.2/HCl 10N) |
| Triton X-100 | 0.1% |

Stock solution of TBS (10X) is prepared, and Triton X-100 is added to the buffer during dilution.
g. HNTG buffer:

| | |
|---|---|
| HEPES | 20 mM |
| NaCl | 150 mM (pH 7.2/HCl 1N) |
| Glycerol | 10% |
| Triton X-100 | 0.2% |

Stock solution (5X) is prepared and kept at 4°C.
h. EDTA/HCl: 0.5 M pH 7.0 (NaOH) as 100X stock.
i. Na₃VO₄: 0.5 M as 100X stock and aliquots are kept in -80°C.
j. Na₄P₂O₇: 0.2 M as 100X stock.
k. Insulin-like growth factor-1 from Promega (Cat# G5111).
l. Rabbit polyclonal anti-phosphotyrosine antiserum.
m. Goat anti-rabbit IgG, POD conjugate (detection antibody), Tago (Cat. No. 4520, Lot No. 1802): Tago, Inc., Burlingame, CA.
n. ABTS (2,2'-azinobis(3-ethylbenzthiazolinesulfonic acid)) solution:

| | |
|---|---|
| Citric acid | 100 mM |
| Na₂HPO₄ | 250 mM (pH 4.0/1 N HCl) |
| ABTS | 0.5 mg/ml |

ABTS solution should be kept in dark and 4°C. The solution should be discarded when it turns green.
o. Hydrogen Peroxide: 30% solution is kept in the dark and at 4°C.

Procedure. All the following steps are conducted at room temperature unless it is specifically indicated. All ELISA plate washings are performed by rinsing the plate with tap water three times, followed by one TBST rinse. Pat plate dry with paper towels.

### A. Cell Seeding:

1. The cells, grown in tissue culture dish (Corning 25020-100) to 80-90% confluence, are harvested with Trypsin-EDTA (0.25%, 0.5 ml/D-100, GIBCO).
2. Resuspend the cells in fresh DMEM + 10% FBS + 2mM L-Glutamine, and transfer to 96-well tissue culture plate (Corning, 25806-96) at 20,000 cells/well (100 µl/well). Incubate for 1 day then replace medium to serum-free medium (90/µl) and incubate in 5% CO₂ and 37°C overnight.

### B. ELISA Plate Coating and Blocking:

1. Coat the ELISA plate (Corning 25805-96) with Anti-IGF-1R Antibody at 0.5 µg/well in 100 µl PBS at least 2 hours.
2. Remove the coating solution, and replace with 100 µl Blocking Buffer, and shake for 30 minutes. Remove the blocking buffer and wash the plate just before adding lysate.

### C. Assay Procedures:

1. The drugs are tested in serum-free condition.
2. Dilute drug stock (in 100% DMSO) 1:10 with DMEM in 96-well poly-propylene plate, and transfer 10 µl/well of this solution to the cells to achieve final drug dilution 1:100, and final DMSO concentration of 1.0%. Incubate the cells in 5% CO₂ at 37°C for 2 hours.
3. Prepare fresh cell lysis buffer (HNTG*)

| | |
|---|---|
| HNTG | 2 ml |
| EDTA | 0.1 ml |
| Na₃VO₄ | 0.1 ml |
| Na₄(P₂O₇) | 0.1 ml |
| H₂O | 7.3 ml |

4. After drug incubation for two hours, transfer 10 µl/well of 200nM IGF-1 Ligand in PBS to the cells (Final Conc. = 20 nM), and incubate at 5% CO₂ at 37°C for 10 minutes.
5. Remove media and add 100µl/well HNTG* and shake for 10 minutes. Look at cells under microscope to see if they are adequately lysed.
6. Use a 12-channel pipette to scrape the cells from the plate, and homogenize the lysate by repeated aspiration and dispensing. Transfer all the lysate to the antibody coated ELISA plate, and shake for 1 hour.
7. Remove the lysate, wash the plate, transfer anti-pTyr (1:3,000 with TBST) 100 µl/well, and shake for 30 minutes.
8. Remove anti-pTyr, wash the plate, transfer TAGO (1:3,000 with TBST) 100 µl/well, and shake for 30 minutes.
9. Remove detection antibody, wash the plate, and transfer fresh ABTS/H₂O₂ (1.2 µl H₂O₂ to 10 ml ABTS) 100 µl/well to the plate to start color development.
10. Measure OD at 410 nm with a reference wavelength of 630 nm in Dynatec MR5000.

### e. EGF Receptor ELISA

EGF Receptor kinase activity in cells genetically engineered to express human EGF-R was measured as described below:
Materials and Reagents. The following materials and reagents were used
a. EGF Ligand: stock concentration = 16.5 µM; EGF 201, TOYOBO, Co., Ltd. Japan.
b. 05-101 (UBI) (a monoclonal antibody recognizing an EGFR extracellular domain).
c. Anti-phosphotyosine antibody (anti-Ptyr) (polyclonal) .
d. Detection antibody: Goat anti-rabbit 1gG horse radish peroxidase conjugate, TAGO, Inc., Burlingame, CA.
e. TBST buffer:

| | |
|---|---|
| Tris-HCl, pH 7 | 50 mM |
| NaCl | 150 mM |
| Triton X-100 | 0.1 |

f. HNTG 5X stock:

| | |
|---|---|
| HEPES | 0.1 M |
| NaCl | 0.75 M |
| Glycerol | 50 |
| Triton X-100 | 1.0% |

g. ABTS stock:

| | |
|---|---|
| Citric Acid | 100 mM |
| Na₂HPO₄ | 250 mM |
| HCl, conc. | 4.0 pH |
| ABTS* | 0.5 mg/ml |

Keep solution in dark at 4°C until used.
h. Stock reagents of:

| | |
|---|---|
| EDTA | 100 mM pH 7.0 |
| Na₃VO₄ | 0.5 M |
| Na₄(P₂O₇) | 0.2 M |

Procedure. The following protocol was used:

### A. Pre-coat ELISA Plate

1. Coat ELISA plates (Corning, 96 well, Cat. #25805-96) with 05-101 antibody at 0.5 µg per well in PBS, 150 µl final volume/well, and store overnight at 4°C. Coated plates are good for up to 10 days when stored at 4°C.
2. On day of use, remove coating buffer and replace with blocking buffer (5% Carnation Instant NonFat Dry Milk in PBS). Incubate the plate, shaking, at room temperature (about 23°C to 25°C) for 30 minutes. Just prior to use, remove blocking buffer and wash plate 4 times with TBST buffer.

### B. Seeding Cells

1. NIH 3T3/C7 cell line (Honegger, et al., Cell 51:199-209, 1987) can be use for this assay.
2. Choose dishes having 80-90% confluence for the experiment. Trypsinize cells and stop reaction by adding 10% CS DMEM medium. Suspend cells in DMEM medium (10% CS DMEM medium) and centrifuge once at 1000 rpm at room temperature for 5 minutes.
3. Resuspend cells in seeding medium (DMEM, 0.5% bovine serum), and count the cells using trypan blue. Viability above 90% is acceptable. Seed cells in DMEM medium (0.5% bovine serum) at a density of 10,000 cells per well, 100 µl per well, in a 96 well microtiter plate. Incubate seeded cells in 5% CO₂ at 37°C for about 40 hours.

### C. Assay Procedures.

1. Check seeded cells for contamination using an inverted microscope. Dilute drug stock (10 mg/ml in DMSO) 1:10 in DMEM medium, then transfer 5 µl to a test well for a final drug dilution of 1:200 and a final DMSO concentration of 1%. Control wells receive DMSO alone. Incubate in 5% CO₂ at 37°C for one hour.
2. Prepare EGF ligand: dilute stock EGF in DMEM so that upon transfer of 10 µl dilute EGF (1:12 dilution), 25 nM final concentration is attained.
3. Prepare fresh 10 ml HNTG* sufficient for 100 µl per well wherein HNTG* comprises: HNTG stock (2.0 ml), milli-Q H₂O (7.3 ml), EDTA, 100 mM, pH 7.0 (0.5 ml), Na₃VO₄ 0.5 M (0.1 ml) and Na₄(P₂O₇), 0.2 M (0.1 ml).
4. Place on ice.
5. After two hours incubation with drug, add prepared EGF ligand to cells, 10 µl per well, to yield a final concentration of 25 nM. Control wells receive DMEM alone. Incubate, shaking, at room temperature, for 5 minutes.
6. Remove drug, EGF, and DMEM. Wash cells twice with PBS. Transfer HNTG* to cells, 100 µl per well. Place on ice for 5 minutes. Meanwhile, remove blocking buffer from other ELISA plate and wash with TBST as described above.
7. With a pipette tip securely fitted to a micropipettor, scrape cells from plate and homogenize cell material by repeatedly aspirating and dispensing the HNTG* lysis buffer. Transfer lysate to a coated, blocked, and washed ELISA plate. Incubate shaking at room temperature for one hour.
8. Remove lysate and wash 4 times with TBST. Transfer freshly diluted anti-Ptyr antibody to ELISA plate at 100 µl per well. Incubate shaking at room temperature for 30 minutes in the presence of the anti-Ptyr antiserum (1:3000 dilution in TBST).
9. Remove the anti-Ptyr antibody and wash 4 times with TBST. Transfer the freshly diluted TAGO 30 anti-rabbit IgG antibody to the ELISA plate at 100 µl per well. Incubate shaking at room temperature for 30 minutes (anti-rabbit IgG antibody: 1:3000 dilution in TBST).
10. Remove detection antibody and wash 4 times with TBST. Transfer freshly prepared ABTS/H₂O₂ solution to ELISA plate, 100 µl per well. Incubate at room temperature for 20 minutes. ABTS/H₂O₂ solution: 1.2 µl 30% H₂O₂ in 10 ml ABTS stock.
11. Stop reaction by adding 50 µl 5N H₂SO₄ (optional), and determine O.D. at 410 nm.
12. The maximal phosphotyrosine signal is determined by subtracting the value of the negative controls from the positive controls. The percent inhibition of phosphotyrosine content for extract-containing wells is then calculated, after subtraction of the negative controls.

### f. Met Autophosphorylation Assay - ELISA

This assay determines Met tyrosine kinase activity by analyzing Met protein tyrosine kinase levels on the Met receptor.

### 1. Reagents

a. HNTG (5X stock solution): Dissolve 23.83 g HEPES and 43.83 g NaCl in about 350 ml dH₂O. Adjust pH to 7.2 with HCl or NaOH, add 500 ml glycerol and 10 ml Triton X-100, mix, add dH₂O to 1 L total volume. To make 1 L of 1X working solution add 200 ml 5X stock solution to 800 ml dH₂O, check and adjust pH as necessary, store at 4°C.
b. PBS (Dulbecco's Phosphate-Buffered Saline), Gibco Cat. # 450-1300EB (1X solution).
c. Blocking Buffer: in 500 ml dH₂O place 100 g BSA, 12.1 g Tris-pH7.5, 58.44 g NaCl and 10 ml Tween-20, dilute to 1 L total volume.
d. Kinase Buffer: To 500 ml dH₂O add 12.1 g TRIS pH7.2, 58.4 g NaCl, 40.7 g MgCl₂ and 1.9 g EGTA; bring to 1 L total volume with dH₂O.
e. PMSF (Phenylmethylsulfonyl fluoride), Sigma Cat. # P-7626, to 435.5 mg, add 100% ethanol to 25 ml total volume, vortex.
f. ATP (Bacterial Source), Sigma Cat. # A-7699, store powder at -20°C; to make up solution for use, dissolve 3.31 mg in 1 ml dH₂O.
g. RC-20H HRPO Conjugated Anti-Phosphotyrosine, Transduction Laboratories Cat. # E120H.
h. Pierce 1-Step (TM) Turbo TMB-ELISA (3,3',5,5'-tetramethylbenzidine, Pierce Cat. # 34022.
i. H₂SO₄ add 1 ml conc. (18N) to 35 ml dH₂O.
j. TRIS HCL, Fischer Cat. # BP152-5; to 121.14 g of material, add 600 ml MilliQ H₂O, adjust pH to 7.5 (or 7.2) with HCl , bring volume to 1 L with MilliQ H₂O.
k. NaCl, Fischer Cat. # S271-10, make up 5M solution.
l. Tween-20, Fischer Cat. # S337-500.
m. Na₃VO₄, Fischer Cat. # S454-50, to 1.8 g material add 80 ml MilliQ H₂O, adjust pH to 10.0 with HCl or NaOH, boil in microwave, cool, check pH, repeat procedure until pH stable at 10.0, add MilliQ H₂O to 100 ml total volume, make 1 ml aliquots and store at -80°C.
n. MgCl₂, Fischer Cat. # M33-500, make up 1M solution.
o. HEPES, Fischer Cat. # BP310-500, to 200 ml MilliQ H₂O, add 59.6 g material, adjust pH to 7.5, bring volume to 250 ml total, sterile filter.
p. Albumin, Bovine (BSA), Sigma Cat. # A-4503, to 30 grams material add sterile distilled water to make total volume of 300 ml, store at 4°C.
q. TBST Buffer: to approx. 900 ml dH₂O in a 1 L graduated cylinder add 6.057 g TRIS and 8.766 g NaCl, when dissolved, adjust pH to 7.2 with HCl, add 1.0 ml Triton X-100 and bring to 1 L total volume with dH₂O.
r. Goat Affinity purified antibody Rabbit IgG (whole molecule), Cappel Cat. # 55641.
s. Anti h-Met (C-28) rabbit polyclonal IgG antibody, Santa Cruz Chemical Cat. # SC-161.
t. Transiently Transfected EGFR/Met chimeric cells (EMR) (Komada, et al., Oncogene, 8:2381-2390 (1993).
u. Sodium Carbonate Buffer, (Na₂CO₄, Fischer Cat. # S495): to 10.6 g material add 800 ml MilliQ H₂O, when dissolved adjust pH to 9.6 with NaOH, bring up to 1 L total volume with MilliQ H₂O, filter, store at 4°C.

### 2. Procedure

All of the following steps are conducted at room temperature unless it is specifically indicated otherwise. All ELISA plate washing is by rinsing 4X with TBST.

### A. EMR Lysis

This procedure can be performed the night before or immediately prior to the start of receptor capture.
1. Quick thaw lysates in a 37°C waterbath with a swirling motion until the last crystals disappear.
2. Lyse cell pellet with 1X HNTG containing 1 mM PMSF. Use 3 ml of HNTG per 15 cm dish of cells. Add 1/□ the calculated HNTG volume, vortex the tube for 1 min., add the remaining amount of HNTG, vortex for another min.
3. Balance tubes, centrifuge at 10,000x g for 10 min at 4°C.
4. Pool supernatants, remove an aliquot for protein determination.
5. Quick freeze pooled sample in dry ice/ethanol bath. This step is performed regardless of whether lysate will be stored overnight or used immediately following protein determination.
6. Perform protein determination using standard bicinchoninic acid (BCA) method (BCA Assay Reagent Kit from Pierce Chemical Cat. # 23225).

### B. ELISA Procedure

1. Coat Corning 96 well ELISA plates with 5 µg per well Goat anti-Rabbit antibody in Carbonate Buffer for a total well volume of 50 µl. Store overnight at 4°C.
2. Remove unbound Goat anti-rabbit antibody by inverting plate to remove liquid.
3. Add 150 µl of Blocking Buffer to each well. Incubate for 30 min. at room temperature with shaking.
4. Wash 4X with TBST. Pat plate on a paper towel to remove excess liquid and bubbles.
5. Add 1µg per well of Rabbit anti-Met antibody diluted in TBST for a total well volume of 100 µl.
6. Dilute lysate in HNTG (90 µg lysate/100µl)
7. Add 100 µl of diluted lysate to each well. Shake at room temperature for 60 min.
8. Wash 4X with TBST. Pat on paper towel to remove excess liquid and bubbles.
9. Add 50 µl of 1X lysate buffer per well.
10. Dilute compounds/extracts 1:10 in 1X Kinase Buffer in a polypropylene 96 well plate.
11. Transfer 5.5 µl of diluted drug to ELISA plate wells. Incubate at room temperature with shaking for 20 min.
12. Add 5.5 µl of 60 µM ATP solution per well. Negative controls do not receive any ATP: Incubate at room temperature for 90 min., with shaking.
13. Wash 4X with TBST. Pat plate on paper towel to remove excess liquid and bubbles.
14. Add 100 µl per well of RC20 (1:3000 dilution in Blocking Buffer). Incubate 30 min. at room temperature with shaking.
15. Wash 4X with TBST. Pat plate on paper towel to remove excess liquid and bubbles.
16. Add 100 µl per well of Turbo-TMB. Incubate with shaking for 30-60 min.
17. Add 100 ul per well of 1M H₂SO₄ to stop reaction.
18. Read assay on Dynatech MR7000 ELISA reader. Test Filter = 450 nm, reference filter = 410 nm.

### g. Biochemical src assay - ELISA

This assay is used to determine src protein kinase activity measuring phosphorylation of a biotinylated peptide as the readout.

### 1. Materials and Reagents:

a. Yeast transformed with src from Courtneidge Laboratory (Sugen, Inc., Redwood City, California).
b. Cell lysates: Yeast cells expressing src are pelleted, washed once with water, re-pelleted and stored at -80°C until use.
c. N-terminus biotinylated EEEYEEYEEEYEEEYEEEY is prepared by standard procedures well known to those skilled in the art.
d. DMSO: Sigma, St. Louis, MO.
e. 96 Well ELISA Plate: Corning 96 Well Easy Wash, Modified flat Bottom Plate, Corning Cat. #25805-96.
f. NUNC 96-well V-bottom polypropylene plates for dilution of compounds: Applied Scientific Cat. # A-72092.
g. Vecastain ELITE ABC reagent: Vector, Burlingame, CA.
h. Anti-src (327) mab: Schizosaccharomyces Pombe was used to express recombinant Src (Superti-Furga, et al., EMBO J., 12:2625-2634; Superti-Furga, et al., Nature Biochem., 14:600-605). S. Pombe strain SP200 (h-s leul.32 ura4 ade210) was grown as described and transformations were pRSP expression plasmids were done by the lithium acetate method (Superti-Furga, supra). Cells were grown in the presence of 1 µM thiamine to repress expression from the nmtl promoter or in the absence of thiamine to induce expression.
i. Monoclonal anti-phosphotyrosine, UBI 05-321 (UB40 may be used instead).
j. Turbo TMB-ELISA peroxidase substrate: Pierce Chemical.

### 2. Buffer Solutions:

a. PBS (Dulbecco's Phosphate-Buffered Saline): GIBCO PBS, GIBCO Cat. # 450-1300EB.
b. Blocking Buffer: 5% Non-fat milk (Carnation) in PBS.
c Carbonate Buffer: Na₂CO₄ from Fischer, Cat. # S495, make up 100 mM stock solution.
d. Kinase Buffer: 1.0 ml (from 1M stock solution) MgCl₂; 0.2 ml (from a 1M stock solution) MnCl₂; 0.2 ml (from a 1M stock solution) DTT; 5.0 ml (from a 1M stock solution) HEPES; 0.1 ml TX-100; bring to 10 ml total volume with MilliQ H₂O.
e. Lysis Buffer: 5.0 HEPES (from 1M stock solution.); 2.74 ml NaCl (from 5M stock solution); 10 ml glycerol; 1.0 ml TX-100; 0.4 ml EDTA (from a 100 mM stock solution); 1.0 ml PMSF (from a 100 mM stock solution); 0.1 ml Na₃VO₄ (from a 0.1 M stock solution); bring to 100 ml total volume with MilliQ H₂O.
f. ATP: Sigma Cat. # A-7699, make up 10 mM stock solution (5.51 mg/ml).
g TRIS-HCl: Fischer Cat. # BP 152-5, to 600 ml MilliQ H₂O add 121.14 g material, adjust pH to 7.5 with HCl, bring to 1 L total volume with MilliQ H₂O.
h. NaCl: Fischer Cat. # S271-10, Make up 5M stock solution with MilliQ H₂O.
   Na₃VO₄: Fischer Cat. # S454-50; to 80 ml MilliQ H₂O, add 1.8 g material; adjust pH to 10.0 with HCl or NaOH; boil in a microwave; cool; check pH, repeat pH adjustment until pH remains stable after heating/cooling cycle; bring to 100 ml total volume with MilliQ H₂O; make 1 ml aliquots and store at -80°C.
j. MgCl₂: Fischer Cat. # M33-500, make up 1M stock solution with MilliQ H₂O.
k. HEPES: Fischer Cat. # BP 310-500; too 200 ml MilliQ H₂O, add 59.6 g material, adjust pH to 7.5, bring to 250 ml total volume with MilliQ H₂O, sterile filter (1M stock solution).
l. TBST Buffer: TBST Buffer: To 900 ml dH₂O add 6.057 g TRIS and 8.766 g NaCl; adjust pH to 7.2 with HCl, add 1.0 ml Triton-X100; bring to 1 L total volume with dH₂O.
m. MnCl₂: Fischer Cat. # M87-100, make up 1M stock solution with MilliQ H₂O.
n. DTT: Fischer Cat. # BP172-5.
o. TBS (TRIS Buffered Saline): to 900 ml MilliQ H₂O add 6.057 g TRIS and 8.777 g NaCl; bring to 1 L total volume with MilliQ H₂O.
p. Kinase Reaction Mixture: Amount per assay plate (100 wells): 1.0 ml Kinase Buffer, 200 µg GST-□ , bring to final volume of 8.0 ml with MilliQ H₂O.
q. Biotin labeled EEEYEEYEEEYEEEYEEEY: Make peptide stock solution (1mM, 2.98 mg/ml) in water fresh just before use.
r. Vectastain ELITE ABC reagent: To prepare 14 ml of working reagent, add 1 drop of reagent A to 15 ml TBST and invert tube several times to mix. Then add 1 drop of reagent B. Put tube on orbital shaker at room temperature and mix for 30 minutes.

### 3. Procedures:

### a. Preparation of src coated ELISA plate.

1. Coat ELISA plate with 0.5µg/well anti-src mab in 100 µl of pH 9.6 sodium carbonate buffer at 4°C overnight.
2. Wash wells once with PBS.
3. Block plate with 0.15 ml 5% milk in PBS for 30 min. at room temperature.
4. Wash plate 5X with PBS.
5. Add 10 µg/well of src transformed yeast lysates diluted in Lysis Buffer (0.1 ml total volume per well). (Amount of lysate may vary between batches.) Shake plate for 20 minutes at room temperature.

### b. Preparation of phosphotyrosine antibody-coated ELISA plate.

1. 4G10 plate: coat 0.5 µg/well 4G10 in 100 µl PBS overnight at 4°C and block with 150 µl of 5% milk in PBS for 30 minutes at room temperature.

### c. Kinase assay procedure.

1. Remove unbound proteins from step 1-7, above, and wash plates 5X with PBS.
2. Add 0.08 ml Kinase Reaction Mixture per well (containing 10 µl of 10X Kinase Buffer and 10 µM (final concentration) biotin-EEEYEEYEEEYEEEYEEEY per well diluted in water.
3. Add 10 µl of compound diluted in water containing 10% DMSO and pre-incubate for 15 minutes at room temperature.
4. Start kinase reaction by adding 10µl/well of 0.05 mM ATP in water (5 µM ATP final).
5. Shake ELISA plate for 15 min. at room temperature.
6. Stop kinase reaction by adding 10 µl of 0.5 M EDTA per well.
7. Transfer 90 µl supernatant to a blocked 4G10 coated ELISA plate from section B, above.
8. Incubate for 30 min. while shaking at room temperature.
9. Wash plate 5X with TBST.
10. Incubate with Vectastain ELITE ABC reagent (100 µl/well) for 30 min. at room temperature.
11. Wash the wells 5X with TBST.
12. Develop with Turbo TMB.

### h. Biochemical lck Assay - ELISA

This assay is used to determine lck protein kinase activities measuring phosphorylation of GST-□ as the readout.

### 1. Materials and Reagents:

a. Yeast transformed with lck. Schizosaccharomyces Pombe was used to express recombinant Lck (Superti-Furga, et al., EMBO J, 12:2625-2634; Superti-Furga, et al., Nature Biotech., 14:600-605). S. Pombe strain SP200 (h-s leul.32 ura4 ade210) was grown as described and transformations with pRSP expression plasmids were done by the lithium acetate method (Superti-Furga, supra). Cells were grown in the presence of 1 µM thiamine to induce expression.
b. Cell lysates: Yeast cells expressing lck are pelleted, washed once in water, re-pelleted and stored frozen at -80°C until use.
c. GST-□: DNA encoding for GST-□ fusion protein for expression in bacteria obtained from Arthur Weiss of the Howard Hughes Medical Institute at the University of California, San Francisco. Transformed bacteria were grown overnight while shaking at 25°C. GST-□ was purified by glutathione affinity chromatography, Pharmacia, Alameda, CA.
d. DMSO: Sigma, St. Louis, MO.
e. 96-Well ELISA plate: Corning 96 Well Easy Wash, Modified Flat Bottom Plate, Corning Cat. #25805-96.
f. NUNC 96-well V-bottom polypropylene plates for dilution of compounds: Applied Scientific Cat. # AS-72092.
g. Purified Rabbit anti-GST antiserum: Amrad Corporation (Australia) Cat. #90001605.
h. Goat anti-Rabbit-IgG-HRP: Amersham Cat. # V010301.
i. Sheep ant-mouse IgG (H+L): Jackson Labs Cat. # 5215-005-003.
j. Anti-Lck (3A5) mab: Santa Cruz Biotechnology Cat # sc-433.
k. Monoclonal anti-phosphotyrosine UBI 05-321 (UB40 may be used instead).

### 2. Buffer solutions:

a. PBS (Dulbecco's Phosphate-Buffered Saline) 1X solution: GIBCO PBS, GIBCO Cat. # 450-1300EB.
b. Blocking Buffer: 100 g. BSA, 12.1 g. TRIS-pH7.5, 58.44 g NaCl, 10 ml Tween-20, bring up to 1 L total volume with MilliQ H₂O.
c. Carbonate Buffer: Na₂CO₄ from Fischer, Cat. # S495; make up 100 mM solution with MilliQ H₂O.
d. Kinase Buffer: 1.0 ml (from 1M stock solution) MgCl₂; 0.2 ml (from a 1M stock solution) MnCl₂; 0.2 ml (from a 1M stock solution) DTT; 5.0 ml (from a 1M stock solution) HEPES; 0.1 ml TX-100; bring to 10 ml total volume with MilliQ H₂O.
e. Lysis Buffer: 5.0 HEPES (from 1M stock solution.); 2.74 ml NaCl (from 5M stock solution); 10 ml glycerol; 1.0 ml TX-100; 0.4 ml EDTA (from a 100 mM stock solution); 1.0 ml PMSF (from a 100 mM stock solution); 0.1 ml Na₃VO₄ (from a 0.1 M stock solution); bring to 100 ml total volume with MilliQ H₂O.
f. ATP: Sigma Cat. # A-7699, make up 10 mM stock solution (5.51 mg/ml).
g TRIS-HCl: Fischer Cat. # BP 152-5, to 600 ml MilliQ H₂O add 121.14 g material, adjust pH to 7.5 with HCl, bring to 1 L total volume with MilliQ H₂O.
h. NaCl: Fischer Cat. # S271-10, Make up 5M stock solution with MilliQ H₂O.
i Na₃VO₄: Fischer Cat. # S454-50; to 80 ml MilliQ H₂O, add 1.8 g material; adjust pH to 10.0 with HCl or NaOH; boil in a microwave; cool; check pH, repeat pH adjustment until pH remains stable after heating/cooling cycle; bring to 100 ml total volume with MilliQ H₂O; make 1 ml aliquots and store at -80°C.
j. MgCl₂: Fischer Cat. # M33-500, make up 1M stock solution with MilliQ H₂O.
k. HEPES: Fischer Cat. # BP 310-500; to 200 ml MilliQ H₂O, add 59.6 g material, adjust pH to 7.5, bring to 250 ml total volume with MilliQ H₂O, sterile filter (1M stock solution).
l. Albumin, Bovine (BSA), Sigma Cat. # A4503; to 150 ml MilliQ H₂O add 30 g material, bring 300 ml total volume with MilliQ H₂O, filter through 0.22 µm filter, store at 4°C.
m. TBST Buffer: To 900 ml dH₂O add 6.057 g TRIS and 8.766 g NaCl; adjust pH to 7.2 with HCl, add 1.0 ml Triton-X100; bring to 1 L total volume with dH₂O.
n. MnCl₂: Fischer Cat. # M87-100, make up 1M stock solution with MilliQ H₂O.
o. DTT; Fischer Cat. # BP172-5.
p. TBS (TRIS Buffered Saline): to 900 ml MilliQ H₂O add 6.057 g TRIS and 8.777 g NaCl; bring to 1 L total volume with MilliQ H₂O.
q Kinase Reaction Mixture: Amount per assay plate (100 wells): 1.0 ml Kinase Buffer, 200 µg GST-□ , bring to final volume of 8.0 ml with MilliQ H₂O.

### 2. Procedures:

### a. Preparation of Lck coated ELISA plate.

1. Coat 2.0 µg/well Sheep anti-mouse IgG in 100 µl of pH 9.6 sodium carbonate buffer at 4°C overnight.
2. Wash well once with PBS.
3. Block plate with 0.15 ml of blocking Buffer for 30 min. at room temp.
4. Wash plate 5X with PBS.
5. Add 0.5 µg/well of anti-lck (mab 3A5) in 0.1 ml PBS at room temperature for 1-2 hours.
6. Wash plate 5X with PBS.
7. Add 20 µg/well of lck transformed yeast lysates diluted in Lysis Buffer (0.1 ml total volume per well). (Amount of lysate may vary between batches) Shake plate at 4°C overnight to prevent loss of activity.

### b. Preparation of phosphotyrosine antibody-coated ELISA plate.

1. UB40 plate: 1.0 µg/well UB40 in 100 µl of PBS overnight at 4°C and block with 150 µl of Blocking Buffer for at least 1 hour.

### c. Kinase assay procedure.

1. Remove unbound proteins from step 1-7, above, and wash plates 5X with PBS.
2. Add 0.08 ml Kinase Reaction Mixture per well (containing 10 µl of 10X Kinase Buffer and 2 µg GST-□ per well diluted with water).
3. Add 10 µl of compound diluted in water containing 10% DMSO and pre-incubate for 15 minutes at room temperature.
4. Start kinase reaction by adding 10µl/well of 0.1 mM ATP in water (10 µM ATP final).
5. Shake ELISA plate for 60 min. at room temperature.
6. Stop kinase reaction by adding 10 µl of 0.5 M EDTA per well.
7. Transfer 90 µl supernatant to a blocked 4G10 coated ELISA plate from section B, above.
8. Incubate while shaking for 30 min. at room temperature.
9. Wash plate 5X with TBST.
10. Incubate with Rabbit anti-GST antibody at 1:5000 dilution in 100 µl TBST for 30 min. at room temperature.
11. Wash the wells 5X with TBST.
12. Incubate with Goat anti-Rabbit-IgG-HRP at 1:20,000 dilution in 100 µl of TBST for 30 min. at room temperature.
13. Wash the wells 5X with TBST.
14. Develop with Turbo TMB.

### i. Assay Measuring Phosphorylating Function Of Raf

The following assay reports the amount of RAF-catalyzed phosphorylation of its target protein MEK as well as MEK's target MAPK. The RAF gene sequence is described in Bonner et al., 1985, Molec. Cell. Biol. 5: 1400-1407, and is readily accessible in multiple gene sequence data banks. Construction of the nucleic acid vector and cell lines utilized for this portion of the invention are fully described in Morrison et al., 1988, Proc. Natl. Acad. Sci. USA 85: 8855-8859.

### Materials and Reagents

1. *Sf9 (Spodoptera frugiperda)* cells; GIBCO-BRL, Gaithersburg, MD.
2. RIPA buffer: 20 mM Tris/HCl pH 7.4, 137 mM NaCl, 10% glycerol, 1 mM PMSF, 5 mg/L Aprotenin, 0.5% Triton X-100;
3. Thioredoxin-MEK fusion protein (T-MEK): T-MEK expression and purification by affinity chromatography were performed according to the manufacturer's procedures. Catalog# K 350-01 and R 350-40, Invitrogen Corp., San Diego, CA
4. His-MAPK (ERK 2); His-tagged MARK was expressed in XL1 Blue cells transformed with pUC18 vector encoding His-MAPK. His-MAPK was purified by Ni-affinity chromatography. Cat# 27-4949-01, Pharmacia, Alameda, CA, as described herein.
5. Sheep anti mouse IgG: Jackson laboratories, West Grove, PA. Catalog, # 515-006-008, Lot# 28563
6. RAF-1 protein kinase specific antibody: URP2653 from UBI.
7. Coating buffer: PBS; phosphate buffered saline, GIBCO-BRL, Gaithersburg, MD
8. Wash buffer: TBST - 50 mM Tris/HCL pH 7.2, 150 mM NaCl, 0.1% Triton X-100
9. Block buffer: TBST, 0.1% ethanolamine pH 7.4
10. DMSO, Sigma, St. Louis, MO
11. Kinase buffer (KB): 20 mM HEPES/HCl pH 7.2, 150 mM NaCl, 0.1% Triton X-100, 1 mM PMSF, 5 mg/L Aprotenin, 75 mM sodium ortho vanadate, 0.5 MM DTT and 10 mM MgCl₂.
12. ATP mix: 100 mM MgCl₂, 300 mM ATP, 10 mCi ³³P ATP (Dupont-NEN)/mL.
13 Stop solution: 1% phosphoric acid; Fisher, Pittsburgh, PA.
14. Wallac Cellulose Phosphate Filter mats; Wallac, Turku, Finnland.
15. Filter wash solution: 1% phosphoric acid, Fisher, Pittsburgh, PA.
16. Tomtec plate harvester, Wallac, Turku, Finnland.
17. Wallac beta plate reader # 1205, Wallac, Turku, Finnland.
18. NUNC 96-well V bottom polypropylene plates for compounds Applied Scientific Catalog # AS-72092.

### Procedure

All of the following steps were conducted at room temperature unless specifically indicated.
1. ELISA plate coating: ELISA wells are coated with 100 ml of Sheep anti mouse affinity purified antiserum (1 mg/100 mL coating buffer) over night at 4°C. ELISA plates can be used for two weeks when stored at 4°C.
2. Invert the plate and remove liquid. Add 100 mL of blocking solution and incubate for 30 min.
3. Remove blocking solution and wash four times with wash buffer. Pat the plate on a paper towel to remove excess liquid.
4. Add 1 mg of antibody specific for RAF-1 to each well and incubate for 1 hour. Wash as described in step 3.
5. Thaw lysates from RAS/RAF infected Sf9 cells and dilute with TBST to 10 mg/100 mL. Add 10 mg of diluted lysate to the wells and incubate for 1 hour. Shake the plate during incubation. Negative controls receive no lysate. Lysates from RAS/RAF infected Sf9 insect cells are prepared after cells are infected with recombinant baculoviruses at a MOI of 5 for each virus, and harvested 48 hours later. The cells are washed once with PBS and lysed in RIPA buffer. Insoluble material is removed by centrifugation (5 min at 10 000 x g). Aliquots of lysates are frozen in dry ice/ethanol and stored at -80°C until use.
6. Remove non-bound material and wash as outlined above (step 3).
7. Add 2 mg of T-MEK and 2 mg of His-MAEPK per well and adjust the volume to 40 mL with kinase buffer. Methods for purifying T-MEK and MAPK from cell extracts are provided herein by example.
8. Pre-dilute compounds (stock solution 10 mg/mL DMSO) or extracts 20 fold in TBST plus 1% DMSO. Add 5 mL of the pre-diluted compounds/extracts to the wells described in step 6. Incubate for 20 min. Controls receive no drug.
9. Start the kinase reaction by addition of 5 mL ATP mix; Shake the plates on an ELISA plate shaker during incubation. 10. Stop the kinase reaction after 60 min by addition of 30 mL stop solution to each well.
11. Place the phosphocellulose mat and the ELISA plate in the Tomtec plate harvester. Harvest and wash the filter with the filter wash solution according to the manufacturers recommendation. Dry the filter mats. Seal the filter mats and place them in the holder. Insert the holder into radioactive detection apparatus and quantify the radioactive phosphorous on the filter mats.

Alternatively, 40 mL aliquots from individual wells of the assay plate can be transferred to the corresponding positions on the phosphocellulose filter mat. After air drying the filters, put the filters in a tray. Gently rock the tray, changing the wash solution at 15 min intervals for 1 hour. Air-dry the filter mats. Seal the filter mats and place them in a holder suitable for measuring the radioactive phosphorous in the samples. Insert the holder into a detection device and quantify the radioactive phosphorous on the filter mats.

### j. CDK2/Cyclin A - Inhibition Assay

This assay analyzes the protein kinase activity of CDK2 in exogenous substrate.

### Reagents:

A: Buffer A (80 mM Tris ( pH 7.2), 40 mM MgCl₂): 4.84 G. Tris (F.W. =121.1 g/mol), 4.07 g. MgCl₂ (F.W.=203.31 g/mol) dissolved in 500 ml H₂O. Adjust pH to 7.2 with HCl.
B. Histone H1 solution (0.45 mg/ml Histone H1 and 20 mM HEPES pH 7.2 (pH 7.4 is OK): 5 mg Histone H1 (Boehinger Mannheim) in 11.111 ml 20 mM HEPES pH 7.2 (477 mg HEPES (F.W.= 238.3 g/mol) dissolved in 100 ml ddH₂O, stored in 1 ml aliquots at -80°C.
C. ATP solution (60 µM ATP, 300 µg/ml BSA, 3 mM DTT): 120 µl 10 mM ATP, 600 µl 10 mg/ml BSA to 20 ml, stored in 1 ml aliquots at -80°C.
D. CDK2 solution: cdk2/cyclin A in 10 mM HEPES pH 7.2, 25 mM NaCl, 0.5 mM DTT, 10% glycerol, stored in 9 µl aliquots at -80°C.

### Description of Assay:

1. Prepare solutions of inhibitors at three times the desired final assay concentration in ddH₂O/15% DMSO by volume.
2. Dispense 20 µl of inhibitors to wells of polypropylene 96-well plates (or 20 µl 15% DMSO for positive and negative controls).
3. Thaw Histone H1 solution (1 ml/plate), ATP solution (1 ml/plate plus 1 aliquot for negative control), and CDK2 solution (9µl/plate). Keep CDK2 on ice until use. Aliquot CDK2 solution appropriately to avoid repeated freeze-thaw cycles.
4. Dilute 9 µl CDK2 solution into 2.1 ml Buffer A (per plate). Mix. Dispense 20 µl into each well.
5. Mix 1 ml Histone H1 solution with 1 ml ATP solution (per plate) into a 10 ml screw cap tube. Add γ³³P ATP to a concentration of 0.15 µCi/20µl (0.15 µCi/well in assay). Mix carefully to avoid BSA frothing. Add 20 µl to appropriate wells. Mix plates on plate shaker. For negative control, mix ATP solution with an equal amount of 20 mM HEPES pH 7.2 and add γ³³P ATP to a concentration of 0.15 µCi/20µl solution. Add 20 µl to appropriate wells.
6. Let reactions proceed for 60 minutes.
7. Add 35 µl 10% TCA to each well. Mix plates on plate shaker.
8. Spot 40 µl of each sample onto P30 filter mat squares. Allow mats to dry (approx. 10-20 minutes).
9 Wash filter mats 4 X 10 minutes with 250 ml 1% phosphoric acid (10 ml phosphoric acid per liter ddH₂O).
10. Count filter mats with beta plate reader.

### 2. Cellular/Biologic Assays

### Assay 1: PDGF-Induced BrdU Incorporation Assay

### Materials and Reagents:

(1) PDGF: human PDGF B/B; 1276-956, Boehringer Mannheim, Germany.
(2) BrdU Labeling Reagent: 10 mM, in PBS (pH7.4), Cat. No. 1 647 229, Boehringer Mannheim, Germany.
(3) FixDenat: fixation solution (ready to use), Cat. No. 1 647 229, Boehringer Mannheim, Germany.
(4) Anti-BrdU-POD: mouse monoclonal antibody conjugated with peroxidase, Cat. No. 1 647 229, Boehringer Mannheim, Germany.
(5) TMB Substrate Solution: tetramethylbenzidine (TMB), ready to use, Cat. No. 1 647 229, Boehringer Mannheim, Germany.
(6) PBS Washing Solution : 1X PBS, pH 7.4, made in house (Sugen, Inc., Redwood City, California).
(7) Albumin, Bovine (BSA): fraction V powder; A-8551, Sigma Chemical Co., USA.
(8) 3T3 cell line genetically engineered to express human PDGF-R.

### Protocol

(1) Cells are seeded at 8000 cells/well in DMEM, 10% CS, 2mM Gln in a 96 well plate. Cells are incubated overnight at 37°C in 5% CO₂.
(2) After 24 hours, the cells are washed with PBS, and then are serum starved in serum free medium (0%CS DMEM with 0.1% BSA) for 24 hours.
(3) On day 3, ligand (PDGF, 3.8 nM, prepared in DMEM with 0.1% BSA) and test compounds are added to the cells simultaneously. The negative control wells receive serum free DMEM with 0.1% BSA only; the positive control cells receive the ligand (PDGF) but no test compound. Test compounds are prepared in serum free DMEM with ligand in a 96 well plate, and serially diluted for 7 test concentrations.
(4) After 20 hours of ligand activation, diluted BrdU labeling reagent (1:100 in DMEM, 0.1% BSA) is added and the cells are incubated with BrdU (final concentration=10 µM) for 1.5 hours.
(5) After incubation with labeling reagent, the medium is removed by decanting and tapping the inverted plate on a paper towel. FixDenat solution is added (50 µl/well) and the plates are incubated at room temperature for 45 minutes on a plate shaker.
(6) The FixDenat solution is thoroughly removed by decanting and tapping the inverted plate on a paper towel. Milk is added (5% dehydrated milk in PBS, 200 µl/well) as a blocking solution and the plate is incubated for 30 minutes at room temperature on a plate shaker.
   The blocking solution is removed by decanting and the wells are washed once with PBS. Anti-BrdU-POD solution (1:100 dilution in PBS, 1% BSA) is added (100 µl/well) and the plate is incubated for 90 minutes at room temperature on a plate shaker.
(8) The antibody conjugate is thoroughly removed by decanting and rinsing the wells 5 times with PBS, and the plate is dried by inverting and tapping on a paper towel.
(9) TMB substrate solution is added (100 µl/well) and incubated for 20 minutes at room temperature on a plate shaker until color development is sufficient for photometric detection.
(10) The absorbance of the samples are measured at 410 nm (in "dual wavelength" mode with a filter reading at 490 nm, as a reference wavelength) on a Dynatech ELISA plate reader.

### Assay 2: EGF-Induced BrdU Incorporation Assay

### Materials and Reagents

(1) EGF: mouse EGF, 201; Toyobo, Co., Ltd. Japan.
(2) BrdU Labeling Reagent: 10 mM, in PBS (pH7.4), Cat. No. 1 647 229, Boehringer Mannheim, Germany.
(3) FixDenat: fixation solution (ready to use), Cat. No. 1 647 229, Boehringer Mannheim, Germany.
(4) Anti-BrdU-POD: mouse monoclonal antibody conjugated with peroxidase, Cat. No. 1 647 229, Boehringer Mannheim, Germany.
(5) TMB Substrate Solution: tetramethylbenzidine (TMB), ready to use, Cat. No. 1 647 229, Boehringer Mannheim, Germany.
(6) PBS Washing Solution : 1X PBS, pH 7.4, made in house (Sugen, Inc., Redwood City, California).
(7) Albumin, Bovine (BSA): fraction V powder; A-8551, Sigma Chemical Co., USA.
(8) 3T3 cell line genetically engineered to express human EGF-R.

### Protocol

(1) Cells are seeded at 8000 cells/well in 10% CS, 2mM Gln in DMEM, in a 96 well plate. Cells are incubated overnight at 37°C in 5% CO₂.
(2) After 24 hours, the cells are washed with PBS, and then are serum starved in serum free medium (0% CS DMEM with 0.1% BSA) for 24 hours.
(3) On day 3, ligand (EGF, 2 nM, prepared in DMEM with 0.1% BSA) and test compounds are added to the cells simultaneously. The negative control wells receive serum free DMEM with 0.1% BSA only; the positive control cells receive the ligand (EGF) but no test compound. Test compounds are prepared in serum free DMEM with ligand in a 96 well plate, and serially diluted for 7 test concentrations.
(4) After 20 hours of ligand activation, diluted BrdU labeling reagent (1:100 in DMEM, 0.1% BSA) is added and the cells are incubated with BrdU (final concentration=10 µM) for 1.5 hours.
(5) After incubation with labeling reagent, the medium is removed by decanting and tapping the inverted plate on a paper towel. FixDenat solution is added (50 µl/well) and the plates are incubated at room temperature for 45 minutes on a plate shaker.
(6) The FixDenat solution is thoroughly removed by decanting and tapping the inverted plate on a paper towel. Milk is added (5% dehydrated milk in PBS, 200 µl/well) as a blocking solution and the plate is incubated for 30 minutes at room temperature on a plate shaker.
(7) The blocking solution is removed by decanting and the wells are washed once with PBS. Anti-BrdU-POD solution (1:100 dilution in PBS, 1% BSA) is added (100 µl/well) and the plate is incubated for 90 minutes at room temperature on a plate shaker.
(8) The antibody conjugate is thoroughly removed by decanting and rinsing the wells 5 times with PBS, and the plate is dried by inverting and tapping on a paper towel.
(9) TMB substrate solution is added (100 µl/well) and incubated for 20 minutes at room temperature on a plate shaker until color development is sufficient for photometric detection.
(10) The absorbance of the samples are measured at 410 nm (in "dual wavelength" mode with a filter reading at 490 nm, as a reference wavelength) on a Dynatech ELISA plate reader.

### Assay 3: EGF-Induced Her2-Driven BrdU Incorporation

### Materials and Reagents:

(1) EGF: mouse EGF, 201; Toyobo, Co., Ltd. Japan
(2) BrdU Labeling Reagent: 10 mM, in PBS (pH7.4), Cat. No. 1 647 229, Boehringer Mannheim, Germany.
(3) FixDenat: fixation solution (ready to use), Cat. No. 1 647 229, Boehringer Mannheim, Germany.
(4) Anti-BrdU-POD: mouse monoclonal antibody conjugated with peroxidase, Cat. No. 1 647 229, Boehringer Mannheim, Germany.
(5) TMB Substrate Solution: tetramethylbenzidine (TMB), ready to use, Cat. No. 1 647 229, Boehringer Mannheim, Germany.
(6) PBS Washing Solution: 1X PBS, pH 7.4, made in house.
(7) Albumin, Bovine (BSA): fraction V powder; A-8551, Sigma Chemical Co., USA.
(8) 3T3 cell line engineered to express a chimeric receptor having the extra-cellular domain of EGF-R and the intra-cellular domain of Her2.

### Protocol:

(1) Cells are seeded at 8000 cells/well in DMEM, 10% CS, 2mM Gln in a 96- well plate. Cells are incubated overnight at 37°C in 5% CO₂.
(2) After 24 hours, the cells are washed with PBS, and then are serum starved in serum free medium (0%CS DMEM with 0.1% BSA) for 24 hours.
(3) On day 3, ligand (EGF=2 nM, prepared in DMEM with 0.1% BSA) and test compounds are added to the cells simultaneously. The negative control wells receive serum free DMEM with 0.1% BSA only; the positive control cells receive the ligand (EGF) but no test compound. Test compounds are prepared in serum free DMEM with ligand in a 96 well plate, and serially diluted for 7 test concentrations.
(4) After 20 hours of ligand activation, diluted BrdU labeling reagent (1:100 in DMEM, 0.1% BSA) is added and the cells are incubated with BrdU (final concentration = 10 µM) for 1.5 hours.
(5) After incubation with labeling reagent, the medium is removed by decanting and tapping the inverted plate on a paper towel. FixDenat solution is added (50 µl/well) and the plates are incubated at room temperature for 45 minutes on a plate shaker.
(6) The FixDenat solution is thoroughly removed by decanting and tapping the inverted plate on a paper towel. Milk is added (5% dehydrated milk in PBS, 200 µl/well) as a blocking solution and the plate is incubated for 30 minutes at room temperature on a plate shaker.
(7) The blocking solution is removed by decanting and the wells are washed once with PBS. Anti-BrdU-POD solution (1:100 dilution in PBS, 1% BSA) is added (100 µl/well) and the plate is incubated for 90 minutes at room temperature on a plate shaker.
(8) The antibody conjugate is thoroughly removed by decanting and rinsing the wells 5 times with PBS, and the plate is dried by inverting and tapping on a paper towel.
(9) TMB substrate solution is added (100 µl/well) and incubated for 20 minutes at room temperature on a plate shaker until color development is sufficient for photometric detection.
(10) The absorbance of the samples are measured at 410 nm (in "dual wavelength" mode with a filter reading at 490 nm, as a reference wavelength) on a Dynatech ELISA plate reader.

### Assay 4: IGF1-Induced BrdU Incorporation Assay

### Materials and Reagents:

(1) IGF1 Ligand: human, recombinant; G511, Promega Corp., USA.
(2) BrdU Labeling Reagent: 10 mM, in PBS (pH7.4), Cat. No. 1 647 229, Boehringer Mannheim, Germany.
(3) FixDenat: fixation solution (ready to use), Cat. No. 1 647 229, Boehringer Mannheim, Germany.
(4) Anti-BrdU-POD: mouse monoclonal antibody conjugated with peroxidase, Cat. No. 1 647 229, Boehringer Mannheim, Germany.
(5) TMB Substrate Solution: tetramethylbenzidine (TMB), ready to use, Cat. No. 1 647 229, Boehringer Mannheim, Germany.
(6) PBS Washing Solution: 1X PBS, pH 7.4, made in house (Sugen, Inc., Redwood City, California).
(7) Albumin, Bovine (BSA): fraction V powder; A-8551, Sigma Chemical Co., USA.
(8) 3T3 cell line genetically engineered to express human IGF-1 receptor. Protocol:

(1) Cells are seeded at 8000 cells/well in DMEM, 10% CS, 2mM Gln in a 96- well plate. Cells are incubated overnight at 37°C in 5% CO₂.
(2) After 24 hours, the cells are washed with PBS, and then are serum starved in serum free medium (0%CS DMEM with 0.1% BSA) for 24 hours.
(3) On day 3, ligand (IGF1=3.3 nM, prepared in DMEM with 0.1% BSA) and test compounds are added to the cells simultaneously. The negative control wells receive serum free DMEM with 0.1% BSA only; the positive control cells receive the ligand (IGF1) but no test compound. Test compounds are prepared in serum free DMEM with ligand in a 96 well plate, and serially diluted for 7 test concentrations.
(4) After 16 hours of ligand activation, diluted BrdU labeling reagent (1:100 in DMEM, 0.1% BSA) is added and the cells are incubated with BrdU (final concentration=10 µM) for 1.5 hours.
(5) After incubation with labeling reagent, the medium is removed by decanting and tapping the inverted plate on a paper towel. FixDenat solution is added (50 µl/well) and the plates are incubated at room temperature for 45 minutes on a plate shaker.
(6) The FixDenat solution is thoroughly removed by decanting and tapping the inverted plate on a paper towel. Milk is added (5% dehydrated milk in PBS, 200 µl/well) as a blocking solution and the plate is incubated for 30 minutes at room temperature on a plate shaker.
(7) The blocking solution is removed by decanting and the wells are washed once with PBS. Anti-BrdU-POD solution (1:100 dilution in PBS, 1% BSA) is added (100 µl/well) and the plate is incubated for 90 minutes at room temperature on a plate shaker.
(8) The antibody conjugate is thoroughly removed by decanting and rinsing the wells 5 times with PBS, and the plate is dried by inverting and tapping on a paper towel.
(9) TMB substrate solution is added (100 µl/well) and incubated for 20 minutes at room temperature on a plate shaker until color development is sufficient for photometric detection.
(10) The absorbance of the samples are measured at 410 nm (in "dual wavelength" mode with a filter reading at 490 nm, as a reference wavelength) on a Dynatech ELISA plate reader.

### g. HUV-EC-C Assay

The following protocol may also be used to measure a compound's activity against PDGF-R, FGF-R, VEGF, aFGF or Flk-1/KDR, all of which are naturally expressed by HUV-EC cells.

### Day 0

1. Wash and trypsinize HUV-EC-C cells (human umbilical vein endothelial cells, (American Type Culture Collection; catalogue no. 1730 CRL). Wash with Dulbecco's phosphate-buffered saline (D-PBS; obtained from Gibco BRL; catalogue no. 14190-029) 2 times at about 1 ml/10 cm² of tissue culture flask. Trypsinize with 0.05% trypsin-EDTA in non-enzymatic cell dissociation solution (Sigma Chemical Company; catalogue no. C-1544). The 0.05% trypsin was made by diluting 0.25% trypsin/1 mM EDTA (Gibco; catalogue no. 25200-049) in the cell dissociation solution. Trypsinize with about 1 ml/25-30 cm² of tissue culture flask for about 5 minutes at 37°C. After cells have detached from the flask, add an equal volume of assay medium and transfer to a 50 ml sterile centrifuge tube (Fisher Scientific; catalogue no. 05-539-6).
2. Wash the cells with about 35 ml assay medium in the 50 ml sterile centrifuge tube by adding the assay medium, centrifuge for 10 minutes at approximately 200 g, aspirate the supernatant, and resuspend with 35 ml D-PBS. Repeat the wash two more times with D-PBS, resuspend the cells in about 1 ml assay medium/15 cm ² of tissue culture flask. Assay medium consists of F12K medium (Gibco BRL; catalogue no. 21127-014) + 0.5% heat-inactivated fetal bovine serum. Count the cells with a Coulter Counter□ Coulter Electronics, Inc.) and add assay medium to the cells to obtain a concentration of 0.8-1.0x10⁵ cells/ml.
3. Add cells to 96-well flat-bottom plates at 100 µl/well or 0.8-1.0x10⁴ cells/well; incubate ∼24h at 37°C, 5% CO₂.

### Day 1

1. Make up two-fold drug titrations in separate 96-well plates, generally 50 µM on down to 0 µM. Use the same assay medium as mentioned in day 0, step 2 above. Titrations are made by adding 90 µl/well of drug at 200 µM (4X the final well concentration) to the top well of a particular plate column. Since the stock drug concentration is usually 20 mM in DMSO, the 200 µM drug concentration contains 2% DMSO.
   Therefore, diluent made up to 2% DMSO in assay medium (F12K + 0.5% fetal bovine serum) is used as diluent for the drug titrations in order to dilute the drug but keep the DMSO concentration constant. Add this diluent to the remaining wells in the column at 60 µl/well. Take 60 µl from the 120 µl of 200 µM drug dilution in the top well of the column and mix with the 60 µl in the second well of the column. Take 60 µl from this well and mix with the 60 µl in the third well of the column, and so on until two-fold titrations are completed. When the next-to-the-last well is mixed, take 60 µl of the 120 µl in this well and discard it. Leave the last well with 60 µl of DMSO/media diluent as a non-drug-containing control. Make 9 columns of titrated drug, enough for triplicate wells each for 1) VEGF (obtained from Pepro Tech Inc., catalogue no. 100-200, 2) endothelial cell growth factor (ECGF) (also known as acidic fibroblast growth factor, or aFGF) (obtained from Boehringer Mannheim Biochemica, catalogue no. 1439 600); or, 3) human PDGF B/B (1276-956, Boehringer Mannheim, Germany) and assay media control. ECGF comes as a preparation with sodium heparin.
2. Transfer 50 µl/well of the drug dilutions to the 96-well assay plates containing the 0.8-1.0x10⁴ cells/100 µl/well of the HUV-EC-C cells from day 0 and incubate ∼2 h at 37°C, 5% CO₂.
3. In triplicate, add 50 µl/well of 80 µg/ml VEGF, 20 ng/ml ECGF, or media control to each drug condition. As with the drugs, the growth factor concentrations are 4X the desired final concentration. Use the assay media from day 0 step 2 to make the concentrations of growth factors. Incubate approximately 24 hours at 37°C, 5% CO₂. Each well will have 50 µl drug dilution, 50 µl growth factor or media, and 100 ul cells, = 200 ul/well total. Thus the 4X concentrations of drugs and growth factors become 1X once everything has been added to the wells.

### Day 2

1. Add ³H-thymidine (Amersham; catalogue no. TRK-686) at 1 µCi/well (10 µl/well of 100 µCi/ml solution made up in RPMI media + 10% heat-inactivated fetal bovine serum) and incubate ∼24 h at 37°C, 5% CO₂. Note: ³H-thymidine is made up in RPMI media because all of the other applications for which we use the ³H-thymidine involve experiments done in RPMI. The media difference at this step is probably not significant. RPMI was obtained from Gibco BRL, catalogue no. 11875-051.

### Day 3

1. Freeze plates overnight at -20°C.

### Day 4

1. Thaw plates and harvest with a 96-well plate harvester (Tomtec Harvester 96^{(R)}) onto filter mats (Wallac; catalogue no. 1205-401); read counts on a Wallac Betaplate⁽™⁾ liquid scintillation counter.

### 3. In Vivo Animal Models

### A. Xenograft Animal Models

The ability of human tumors to grow as xenografts in athymic mice (*e*.*g*., Balb/c, nu/nu) provides a useful in vivo model for studying the biological response to therapies for human tumors. Since the first successful xenotransplantation of human tumors into athymic mice, (Rygaard and Povlsen, 1969, Acta Pathol. Microbial. Scand. 77:758-760), many different human tumor cell lines (e.g., mammary, lung, genitourinary, gastro-intestinal, head and neck, glioblastoma, bone, and malignant melanomas) have been transplanted and successfully grown in nude mice. The following assays may be used to determine the level of activity, specificity and effect of the different compounds of the present invention. Three general types of assays are useful for evaluating compounds: cellular/catalytic, cellular/biological and in vivo. The object of the cellular/catalytic assays is to determine the effect of a compound on the ability of a TK to phosphorylate tyrosines on a known substrate in a cell. The object of the cellular/biological assays is to determine the effect of a compound on the biological response stimulated by a TK in a cell. The object of the in vivo assays is to determine the effect of a compound in an animal model of a particular disorder such as cancer.

Suitable cell lines for subcutaneous xenograft experiments include C6 cells (glioma, ATCC # CCL 107), A375 cells (melanoma, ATCC # CRL 1619), A431 cells (epidermoid carcinoma, ATCC # CRL 1555), Calu 6 cells (lung, ATCC # HTB 56), PC3 cells (prostate, ATCC # CRL 1435), SKOV3TP5 cells and NIH 3T3 fibroblasts genetically engineered to overexpress EGFR, PDGFR, IGF-1R or any other test kinase. The following protocol can be used to perform xenograft experiments:

Female athymic mice (BALB/c, nu/nu) are obtained from Simonsen Laboratories (Gilroy, CA). All animals are maintained under clean-room conditions in Micro-isolator cages with Alpha-dri bedding. They receive sterile rodent chow and water ad libitum.

Cell lines are grown in appropriate medium (for example, MEM, DMEM, Ham's F10, or Ham's F12 plus 5% - 10% fetal bovine serum (FBS) and 2 mM glutamine (GLN)). All cell culture media, glutamine, and fetal bovine serum are purchased from Gibco Life Technologies (Grand Island, NY) unless otherwise specified. All cells are grown in a humid atmosphere of 90-95% air and 5-10% CO₂ at 37°C. All cell lines are routinely subcultured twice a week and are negative for mycoplasma as determined by the Mycotect method (Gibco).

Cells are harvested at or near confluency with 0.05% Trypsin-EDTA and pelleted at 450 x g for 10 min. Pellets are resuspended in sterile PBS or media (without FBS) to a particular concentration and the cells are implanted into the hindflank of the mice (8 - 10 mice per group, 2 - 10 x 10⁶ cells/animal). Tumor growth is measured over 3 to 6 weeks using venier calipers. Tumor volumes are calculated as a product of length x width x height unless otherwise indicated. P values are calculated using the Students t-test. Test compounds in 50 - 100 µL excipient (DMSO, or VPD:D5W) was delivered by IP injection at different concentrations generally starting at day one after implantation.

### B. Tumor Invasion Model

The following tumor invasion model has been developed and maybe used for the evaluation of therapeutic value and efficacy of the compounds identified to selectively inhibit KDR/FLK-1 receptor.

### Procedure

8 week old nude mice (female) (Simonsen Inc.) were used as experimental animals. Implantation of tumor cells was performed in a laminar flow hood. For anesthesia, Xylazine/Ketamine Cocktail (100 mg/kg ketamine and 5 mg/kg Xylazine) are administered intraperitoneally. A midline incision is done to expose the abdominal cavity (approximately 1.5 cm in length) to inject 10⁷ tumor cells in a volume of 100 µl medium. The cells are injected either into the duodenal lobe of the pancreas or under the serosa of the colon. The peritoneum and muscles are closed with a 6-0 silk continuous suture and the skin was closed by using wound clips. Animals were observed daily.

### Analysis

After 2-6 weeks, depending on gross observations of the animals, the mice are sacrificed, and the local tumor metastases, to various organs (lung, liver, brain, stomach, spleen, heart, muscle) are excised and analyzed (measurements of tumor size, grade of invasion, immunochemistry, and in situ hybridization).

### D. Measurement of Cell Toxicity

Therapeutic compounds should be more potent in inhibiting protein kinase activity than in exerting a cytotoxic effect. A measure of the effectiveness and cell toxicity of a compound can be obtained by determining the therapeutic index: IC₅₀/LD₅₀. IC₅₀, the dose required to achieve 50% inhibition, can be measured using standard techniques such as those described herein. LD₅₀, the dosage which results in 50% toxicity, can also be measured by standard techniques (Mossman, 1983, J. Immunol. Methods, 65:55-63), by measuring the amount of LDH released (Korzeniewski and Callewaert, 1983, J. Immunol. Methods, **64**:313; Decker and Lohmann-Matthes, 1988, J. Immunol. Methods, 115:61), or by measuring the lethal dose in animal models. Compounds with a large therapeutic index are preferred. The therapeutic index should be greater than 2, preferably at least 10, more preferably at least 50.

### Conclusion

Thus, it will be appreciated that the compounds, and pharmacological compositions of the present invention are effective in modulating PK activity and therefore are expected to be effective as therapeutic agents against RTK, CTK-, and STK-related disorders.

## Claims

1. A 2-indolinone having the chemical structure: 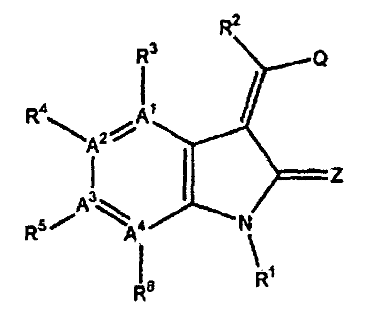 wherein,
A² and A⁴ are nitrogen and A¹ and A³ are carbon or A¹ and A³ are nitrogen and A² and A⁴ are carbon, it being understood that when A¹, A², A³ and A⁴ is nitrogen, R³, R⁴, R⁵ and R⁶, respectively does not exist;
R¹ is selected from the group consisting of hydrogen; C1-C10 alkyl, cycloalkyl, C2-C10 alkenyl, C2-C10 alkynyl, aryl, heteroaryl, trihalomethanecarbonyl, heteroalicyclic, hydroxy, C1-C10 alkoxy, C-carboxy, O-carboxy, C-amido, C-thioamido, guanyl, guanadinyl, ureidyl, sulfonyl, and trihalomethanesulfonyl;
R² is selected from the group consisting of hydrogen, C1-C10 alkyl, cycloalkyl, aryl, and halo;
R³, R⁴ R⁵ and R⁶ are independently selected from the group consisting of hydrogen, C1-C10 alkyl, trihalomethyl, cycloalkyl, C2-C10 alkenyl, C2-C10 alkynyl, aryl, heteroaryl, heteroalicyclic, hydroxy, thiohydroxy, C1-C10 alkoxy, aryloxy, thiohydroxy, C1-C10 thioalkoxy, thioaryloxy, sulfinyl, sulfonyl, S-sulfonamido, N-sulfonamido, N-trihalomethanesulfonamido, carbonyl, aldehyde, trihalomethyl-carbonyl, C-carboxy, O-carboxy, cyano, nitro, halo, cyanato, isocyanato, thiocyanato, isothiocyanato, O-carbamyl, N-carbamyl, O-thiocarbamyl, N-thiocarbamyl, phosphonyl, guanyl, guanidinyl, ureidyl, C-amido, N-amido, amino, quaternary ammonium, -NR¹⁸R¹⁹;
R¹⁸ and R¹⁹ are independently selected from the group consisting of hydrogen, C1-C10 alkyl, cycloalkyl, aryl, carbonyl, acetyl, trihalomethylcarbonyl, C-carboxy, trihalomethanesulfonyl, sulfonyl, C-peptidyl and, combined, a five-member or a six-member heteroalicylic ring;
Z is oxygen;
Q is 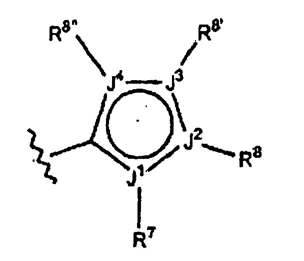 J¹ is selected from the group consisting of oxygen, nitrogen and sulfur;
J², J³ and J⁴ are independently selected from the group consisting of carbon, nitrogen, oxygen and sulfur such that the five-member heteroaryl ring is one known in the chemical arts, it being further understood that, when J², J³ or J⁴ is nitrogen, oxygen or sulfur, R⁸, R^{8'} or R^{8"} respectively, do not exist; likewise when J¹ is oxygen or sulfur, R⁷ does not exist;
R⁷ is selected from the group consisting of hydrogen, C1-C10 alkyl, cycloalkyl, trihalomethylcarbonyl, aryl, guanyl, carboxyl, sulphonyl, and trihalomethanesulfonyl;
one of R⁸, R^{8'} or R^{8"} is H or an - (alk₁) rM group; wherein (alk₁) is CH₂; r is 1 to 3, inclusive; and M is selected from the group consisting of hydroxy, amino, carboxy, morpholinyl, piperazinyl, tetrazolyl, N-carbamyl, O-carbamyl, S-sulfonamido, N-sulfonamido, sulfonyl, -S(O)₂OH and phosphonyl;
the remaining two of R⁸, R^{8'} and R^{8"} are independently selected from the group consisting of:
hydrogen;
unsubstituted C1-C4 alkyl;
C1-C4 alkyl substituted with one or more groups selected from the group consisting of: halo, hydroxyl, unsubstituted C1-C4 alkoxy, amino, s-sulfonamido, -NR¹⁸R¹⁹ or carboxy;
unsubstituted C1-C4 alkoxy;
C1-C4 alkoxy substituted with one or more halogens;
unsubstituted aryl;
aryl substituted with one or more groups selected from the group consisting of unsubstituted C1-C4 alkyl, unsubstituted C1-C4 alkoxy, halo, trihalomethyl, amino, -NR¹⁸R¹⁹, hydroxy or S-sulfonamido;
unsubstituted heteroaryl;
heteroaryl substituted with one or more groups selected from unsubstituted C1-C4 alkyl, unsubstituted C1-C4 alkoxy, halo, trihalomethyl, amino, -NR¹⁸R¹⁹, hydroxy or S-sulfonamido;
unsubstituted heteroalicyclic;
heteroalicyclic substituted with one or more groups selected from unsubstituted C1-C4 alkyl, halo, hydroxy, unsubstituted alkoxy, amino or -NR¹⁸R¹⁹;
halo;
cyano;
carboxy; and
a six member cycloalkyl, aryl, heteroaryl or heteroalicyclic ring formed by combining R⁸ and R^{8'} or R^{8'} and R^{8''};
and physiologically acceptable salts thereof; and wherein:
C1-C10 alkyl is optionally substituted with unsubstituted cycloalkyl selected from the group consisting of cyclopropane, cyclobutane, cyclopentane, cyclopentene, cyclohexane, adamantane, cyclohexadiene, cycloheptane and cycloheptatriene; unsubstituted aryl selected from the group consisting of phenyl, naphthalenyl and anthracenyl; unsubstituted heteroaryl selected from the group consisting of pyrrole, furan, thiophene, imidazole, oxazole, thiazole, pyrazole, pyridine, pyrimidine, quinoline, isoquinoline, purine and carbazole; unsubstituted heteroalicyclic selected from the group consisting of piperidine, morpholine, piperazine, pyrroline, pyrrolidine, dihydrothiophene and tetrahydrofuran; hydroxy; C1-C4 alkoxy, aryloxy; thiohydroxy; C1-C4 thioalkoxy; thioaryloxy; cyano; halo; carbonyl; thiocarbonyl; O-carbamyl; N-carbamyl; O-thiocarbamyl; N-thiocarbamyl; C-amido; N-amido; C-carboxy; O-carboxy; cyanato; isocyanato; thiocyanato; isothiocyanato; nitro; silyl; amino and -NR¹⁸R¹⁹, where R¹⁸ and R¹⁹ are independently selected from the group consisting of hydrogen, unsubstituted C1-C4 alkyl, unsubstituted cycloalkyl as defined above, unsubstituted aryl as defined above, carbonyl, trihalomethylcarbonyl, C-carboxy, trihalomethylsulfonyl, sulfonyl and, combined, a five-member or six-member heteroalicyclic ring as defined above;
cycloalkyl is selected from the group consisting of cyclopropane, cyclobutane, cyclopentane, cyclopentene, cyclohexane, adamantane, cyclohexadiene, cycloheptane and cycloheptatriene, and is optionally substituted with unsubstituted C1-C4 alkyl; unsubstituted aryl as defined above; unsubstituted heteroaryl as defined above; unsubstituted heteroalicyclic as defined above; hydroxy; C1-C4 alkoxy; aryloxy; thiohydroxy; C1-C4 thioalkoxy; thioaryloxy; cyano; halo; carbonyl; thiocarbonyl; carboxy; O-carbamyl; N-carbamyl; C-amido; N-amido; nitro; amino and -NR¹⁸R¹⁹, with R¹⁸ and R¹⁹ as defined above;
C2-C10 alkenyl is optionally substituted as defined for C1-C10 alkyl above;
C2-C10 alkynyl is optionally substituted as defined for C1-C10 alkyl above;
aryl is selected from the group consisting of phenyl, naphthalenyl and anthracenyl, and is optionally substituted with halo; trihalomethyl; unsubstituted C1-C4 alkyl; unsubstituted aryl as defined above; unsubstituted heteroaryl as defined above; unsubstituted heteroalicyclic as defined above; hydroxy; C1-C4 alkoxy, aryloxy; thiohydroxy; C1-C4 thioalkoxy; thioaryloxy; cyano; nitro; carbonyl; thiocarbonyl; C-carboxy; O-carboxy; O-carbamyl; N-carbamyl; O-thiocarbamyl; N-thiocarbamyl; C-amido; N-amido; sulfinyl; sulfonyl; S-sulfonamido; N-sulfonamido; trihalomethanesulfonamido; amino and -NR¹⁸R¹⁹, R¹⁸ and R¹⁹ as defined above;
heteroaryl is selected from the group consisting of pyrrole, furan, thiophene, imidazole, oxazole, thiazole, pyrazole, pyridine, pyrimidine, quinoline, isoquinoline, purine and carbazole, and is optionally substituted with unsubstituted C1-C4 alkyl; unsubstituted cycloalkyl as defined above; unsubstituted aryl as defined above; halo; trihalomethyl; hydroxy; C1-C4 alkoxy, aryloxy; thiohydroxy; C1-C4 thioalkoxy; thioaryloxy; cyano; nitro; carbonyl; thiocarbonyl; sulfonamido; carboxy; sulfinyl; sulfonyl; O-carbamyl; N-carbamyl; O-thiocarbamyl; N-thiocarbamyl; C-amido; N-amido; amino and -NR¹⁸R¹⁹, with R¹⁸ and R¹⁹ as defined above;
heteroalicyclic is selected from the group consisting of piperidine, morpholine, piperazine, pyrroline, pyrrolidine, dihydrothiophene and tetrahydrofuran, and is optionally substituted with unsubstituted C1-C4 alkyl; unsubstituted cycloalkyl as defined above; halo; trihalomethyl; hydroxy; C1-C4 alkoxy, aryloxy; thiohydroxy; C1-C4 thioalkoxy; thioaryloxy; cyano; nitro; carbonyl; thiocarbonyl; carboxy; O-carbamyl; N-carbamyl; O-thiocarbamyl; N-thiocarbamyl; sulfinyl; sulfonyl; C-amido; N-amido; amino and -NR¹⁸R¹⁹, with R¹⁸ and R¹⁹ as defined above.

2. The compound or salt of claim 1 wherein R¹ is hydrogen.

3. The compound or salt of claim 1 wherein R² is hydrogen.

4. The compound or salt of claim 1 wherein R⁷ is hydrogen.

5. The compound or salt of any of claims 1 to 4 wherein R³, R⁴, R⁵ and R⁶ are independently selected from the group consisting of:
hydrogen;
unsubstituted C1-C4 alkyl;
C1-C4 alkyl substituted with a group selected from the group consisting of unsubstituted cycloalkyl, halo, hydroxy, unsubstituted C1-C4 alkoxy, C-carboxy, unsubstituted aryl, unsubstituted heteroaryl, unsubstituted heteroalicyclic, amino, quaternary ammonium or -NR¹⁸NR¹⁹;
unsubstituted cycloalkyl;
hydroxy;
unsubstituted C1-C4 alkoxy;
C1-C4 alkoxy substituted with a group selected from the group consisting of one or more halo groups, unsubstituted aryl or unsubstituted heteroaryl;
trihalomethyl;
halo;
unsubstituted aryl;
aryl substituted with one or more groups independently selected from the group consisting of unsubstituted C1-C4 alkyl, C1-C4 alkyl substituted with one or more halo groups, trihalomethyl, halo, hydroxy, or unsubstituted C1-C4 alkoxy;
unsubstituted aryloxy;
aryloxy substituted with one or more groups independently selected from the group consisting of halo, unsubstituted C1-C4 alkyl, C1-C4 alkyl substituted with one or more halo groups, unsubstituted aryl, hydroxy, unsubstituted C1-C4 alkoxy, amino or -NR¹⁸NR¹⁹;
S-sulfonamido;
unsubstituted heteroaryl;
heteroaryl substituted with one or more groups independently selected from the group consisting of hydrogen, unsubstituted C1-C4 alkyl, C1-C4 alkyl sustituted with one or more halo groups, trihalomethyl, halo, hydroxy, unsubstituted C1-C4 alkoxy, unsubstituted aryloxy, amino, S-sulfonamido or -NR¹⁸R¹⁹;
unsubstituted heteroalicyclic;
heteroalicyclic substituted with one or more groups independently selected from the group consisting of unsubstituted C1-C4 alkyl, C1-C4 alkyl substituted with one or more halo groups, unsubstituted C1-C4 alkyl alkoxy, hydroxy, amino, S-sulfonamido or -NR¹⁸R¹⁹;
unsubstituted C1-C4 alkyl C-carboxy;
carboxylic acid;
unsubstituted C1-C4 alkyl carbonyl;
aldehyde;
unsubstituted aryl carbonyl;
acetyl;
S-sulfonamido;
N-sulfonamido;
amino; and,
-NR¹⁸NR¹⁹.

6. The compound or salt of any of claims 1 to 5 wherein any two of R⁸, R^{8'} or R^{8"} are - (alk₁)rM groups.

7. The compound or salt of any of claims 1 to 6 wherein J¹ is selected from the group consisting of nitrogen, oxygen and sulphur and J², J³ and J⁴ are carbon.

8. The compound or salt of any of claims 1 to 6 wherein J¹ and J³ are nitrogen and J² and J⁴ are carbon.

9. A pharmaceutical composition comprising a compound or salt according to any of claims 1 to 8 and a pharmaceutically acceptable carrier or diluent.

10. The use of a compound according to any of claims 1 to 8 in the manufacture of a medicament for the treatment of a disease selected from a cancer selected from the group consisting of squamous cell carcinoma, astrocytoma, glioblastoma, breast cancer, glioma, melanoma, lung cancer, Small-cell lung cancer, bladder cancer, ovarian cancer, prostate cancer, and head and neck cancer; a proliferation disorder selected from the group consisting of restinosis, fibrosis, psoriasis, osteoarthritis and rheumatoid arthritis; diabetes, autoimmune disease, an inflammatory disorder and angiogenesis.

## Patentansprüche

1. Ein 2-Indolinon mit der chemischen Struktur: 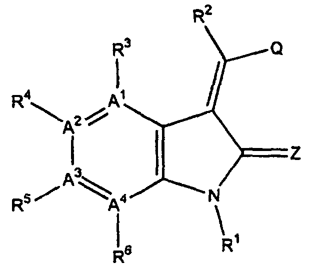 wobei
A² und A⁴ Stickstoff und A¹ und A³ Kohlenstoff oder A¹ und A³ Stickstoff und A² und A⁴ Kohlenstoff sind, wobei zu verstehen ist, dass wenn A¹, A², A³ und A⁴ Stickstoff ist, R³, R⁴, R⁵ bzw. R⁶ nicht vorhanden sind;
R¹ aus der Gruppe, die aus Wasserstoff, C1-C10 Alkyl, Cycloalkyl, C2-C10 Alkenyl, C2-C10 Alkinyl, Aryl, Heteroaryl, Trihalomethancarbonyl, Heteroalicyclen, Hydroxy, C1-C10 Alkoxy, C-Carboxy, O-Carboxy, C-Amido, C-Thioamido, Guanyl, Guanidinylyl, Ureidylyl, Sulfonyl und Trihalogenmethansulfonyl besteht, ausgewählt wird.
R² aus der Gruppe, die aus Wasserstoff, C1-C10 Alkyl, Cycloalkyl, Aryl und Halogen besteht, ausgewählt wird;
R³, R⁴, R⁵ und R⁶ unabhängig voneinander aus der Gruppe, die aus Wasserstoff, C1-C10 Alkyl, Trihalogenmethyl, Cycloalkyl, C2-C10 Alkenyl, C2-C10 Alkinyl, Aryl, Heteroaryl, Heteroalicyclen, Hydroxy, Thiohydroxy, C1-C10 Alkoxy, Aryloxy, Thiohydroxy, C1-C10 Thioalkoxy, Thioaryloxy, Sulfinyl, Sulfonyl, S-Sulfonamido, N-Sulfonamido, N-Trihalogenmethansulfonamido, Carbonyl, Aldehyd, Trihalogenmethylcarbonyl, C-Carboxy, O-Carboxy, Cyano, Nitro, Halogen, Cyanato, Isocyanato, Thiocyanato, Isothiocyanato, O-Carbamyl, N-Carbamyl, O-Thiocarbamyl, N-Thiocarbamyl, Phosphonyl, Guanyl, Guanidinylyl, Ureidylyl, C-Amido, N-Amido, Amino, quartäres Ammonium und -NR¹⁸R¹⁹ besteht, ausgewählt werden.
R¹⁸ und R¹⁹ unabhängig aus der Gruppe, die aus Wasserstoff, C1-C10 Alkyl, Cycloalkyl, Aryl, Carbonyl, Acetyl, Trihalogenmethylcarbonyl, C-Carboxy, Trihalogenmethansulfonyl, Sulfonyl, C-Peptidyl und, kombiniert, einem fünf- oder sechsgliedrigen heteroalicylischen Ring besteht, ausgewählt werden;
Z Sauerstoff ist;
Q 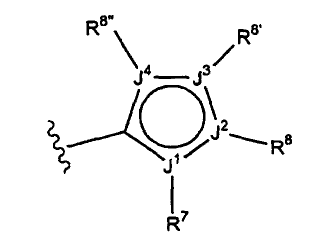 ist;
J¹ aus der Gruppe, die aus Sauerstoff, Stickstoff und Schwefel besteht, ausgewählt wird;
J², J³ und J⁴ unabhängig aus der Gruppe, die aus Kohlenstoff, Stickstoff, Sauerstoff und Schwefel besteht, ausgewählt werden, so dass der fünfgliedrige Heteroarylring eine in der Chemie bekannte Verbindung ist, und es des Weiteren zu verstehen ist, dass, wenn J², J³ oder J⁴ Stickstoff, Sauerstoff oder Schwefel sind, R⁸, R^{8'} bzw. R^{8''} nicht existieren; gleichermaßen existiert R⁷ nicht, wenn J¹ Sauerstoff oder Schwefel ist;
R⁷ aus der Gruppe, die aus Wasserstoff, C1-C10 Alkyl, Cycloalkyl, Trihalogenmethylcarbonyl, Aryl, Guanyl, Carboxyl, Sulfonyl und Trihalogenmethansulfonyl besteht, ausgewählt wird;
einer von R⁸, R^{8'} oder R^{8"} H oder eine -(alk₁)rM-Gruppe ist, wobei (alk₁) CH₂ ist; r 1 bis einschließlich 3 ist; und M aus der Gruppe, die aus Hydroxy, Amino, Carboxy, Morpholinyl, Piperazinyl, Tetrazolyl, N-Carbamyl, O-Carbamyl, S-Sulfonamido, N-Sulfonamido, Sulfonyl, -S(O)₂OH und Phosphonyl besteht, ausgewählt wird;
die übrigen beiden von R⁸, R^{8'} und R^{8''} unabhängig ausgewählt werden aus der Gruppe, die besteht aus:
Wasserstoff;
unsubstituiertem C1-C4 Alkyl;
C1-C4 Alkyl substituiert mit einer oder mehr Gruppen ausgewählt aus der Gruppe bestehend aus: Halogen, Hydroxyl, unsubstituiertem C1-C4 Alkoxy, Amino, S-Sulfonamido, -NR¹⁸R¹⁹ oder Carboxy;
unsubstituiertem C1-C4 Alkoxy;
C1-C4 Alkoxy substituiert mit einem oder mehreren Halogenen;
unsubstituiertem Aryl;
Aryl substituiert mit einer oder mehr Gruppen ausgewählt aus der Gruppe bestehend aus unsubstituiertem C1-C4 Alkyl, unsubstituiertem C1-C4 Alkoxy, Halogen, Trihalogenmethyl, Amino, -NR¹⁸R¹⁹, Hydroxy oder S-Sulfonamido;
unsubstituiertem Heteroaryl;
Heteroaryl substituiert mit einer oder mehr Gruppen ausgewählt aus unsubstituiertem C1-C4 Alkyl, unsubstituiertem C1-C4 Alkoxy, Halogen, Trihalogenmethyl, Amino, -NR¹⁸R¹⁹, Hydroxy oder S-Sulfonamido;
unsubstituierten Heteroalicyclen;
Heteroalicyclen substituiert mit einer oder mehr Gruppen ausgewählt aus unsubstituiertem C1-C4 Alkyl, Halogen, Hydroxy, unsubstituiertem Alkoxy, Amino oder -NR¹⁸R¹⁹;
Halogen;
Cyano;
Carboxy; und
einem, durch die Kombination von R⁸ und R^{8'} oder R^{8'} und R^{8''} gebildeten, sechsgliedrigen Cycloalkyl-, Aryl-, Heteroaryl- oder heteroalicyclischen Ring;
und physiologisch annehmbare Salze davon;
und wobei:
C1-C10 Alkyl optional mit unsubstituiertem Cycloalkyl, das aus der Gruppe, die aus Cyclopropan, Cyclobutan, Cyclopentan, Cyclopenten, Cyclohexan, Adamantan, Cyclohexadien, Cycloheptan und Cycloheptatrien besteht, ausgewählt wird; unsubstituiertem Aryl, das aus der Gruppe, die aus Phenyl, Naphthalenyl, und Anthracenyl besteht, ausgewählt wird; unsubstituiertem Heteroaryl, das aus der Gruppe, die aus Pyrrol, Furan, Thiophen, Imidazol, Oxazol, Thiazol, Pyrazol, Pyridin, Pyrimidin, Chinolin, Isochinolin, Purin und Carbazol besteht, ausgewählt wird; unsubstituierte Heteroalicyclen, die aus der Gruppe, die aus Piperidin, Morpholin, Piperazin, Pyrrolin, Pyrrolidin, Dihydrothiophen und Tetrahydrofuran besteht, ausgewählt werden; Hydroxy; C1-C4 Alkoxy; Aryloxy; Thiohydroxy; C1-C4 Thioalkoxy; Thioaryloxy; Cyano; Halogen; Carbonyl; Thiocarbonyl; O-Carbamyl; N-Carbamyl; O-Thiocarbamyl; N-Thiocarbamyl; C-Amido; N-Amido; C-Carboxy, O-Carboxy, Cyanato; Isocyanato; Thiocyanato; Isothiocyanato; Nitro; Silyl; Amino und NR¹⁸R¹⁹, wobei R¹⁸ und R¹⁹ unabhängig voneinander aus der Gruppe, die aus Wasserstoff, unsubstituiertem C1-C4 Alkyl, unsubstituiertem Cycloalkyl wie oben definiert, unsubstituiertem Aryl wie oben definiert, Carbonyl, Trihalogenmethylcarbonyl, C-Carboxy, Trihalogenmethylsulfonyl, Sulfonyl und kombiniert einem fünfgliedrigen oder sechsgliedrigen heteroalicyclischen Ring wie oben definiert, besteht, ausgewählt wird, substituiert ist;
Cycloalkyl aus der Gruppe, die aus Cyclopropan, Cyclobutan, Cyclopentan, Cyclopenten, Cyclohexan, Adamantan, Cyclohexadien, Cycloheptan und Cycloheptatrien besteht, ausgewählt wird und optional mit unsubstituiertem C1-C4 Alkyl; unsubstituiertem Aryl wie oben definiert; unsubstituiertem Heteroaryl wie oben definiert; unsubstituiertem Heteroalicyclen wie oben definiert; Hydroxy; C1-C4 Alkoxy; Aryloxy; Thiohydroxy; C1-C4 Thioalkoxy; Thioaryloxy; Cyano; Halo; Carbonyl; Thiocarbonyl; Carboxy; O-Carbamyl; N-Carbamyl; C-Amido; N-Amido; Nitro; Amino und -NR¹⁸R¹⁹, mit R¹⁸ und R¹⁹ wie oben definiert, substituiert ist;
C2-C10 Alkenyl optional wie oben für C1-C10 Alkyl definiert substituiert ist;
C2-C10 Alkinyl optional wie oben für C1-C10 Alkyl definiert substituiert ist;
Aryl aus der Gruppe, die aus Phenyl, Naphthalenyl und Anthracenyl besteht, ausgewählt wird und optional mit Halogen, Trihalogenmethyl, unsubstituiertem C1-C4 Alkyl, unsubstituiertem Aryl wie oben definiert, unsubstituiertem Heteroaryl wie oben definiert, unsubstituiertem Heteroalicyclen wie oben definiert, Hydroxy, C1-C4 Alkoxy, Aryloxy, Thiohydroxy, C1-C4 Thioalkoxy, Thioaryloxy, Cyano, Nitro, Carbonyl, Thiocarbonyl, C-Carboxy, O-Carboxy, O-Carbamyl, N-Carbamyl, O-Thiocarbamyl, N-Thiocarbamyl, C-Amido, N-Amido, Sulfinyl, Sulfonyl, S-Sulfonamido, N-Sulfonamido, Trihalogenmethansulfonamido, Amino und -NR¹⁸R¹⁹, mit R¹⁸ und R¹⁹ wie oben definiert, substituiert ist;
Heteroaryl aus der Gruppe, die Pyrrol, Furan, Thiophen, Imidazol, Oxazol, Thiazol, Pyrazol, Pyridin, Pyrimidin, Chinolin, Isochinolin, Purin und Carbazol besteht, ausgewählt wird und optional mit unsubstituiertem C1-C4 Alkyl, unsubstituiertem Cycloalkyl wie oben definiert, unsubstituiertem Aryl wie oben definiert, Halogen, Trihalogenmethyl, Hydroxy, C1-C4 Alkoxy, Aryloxy, Thiohydroxy, C1-C4 Thioalkoxy, Thioaryloxy, Cyano, Nitro, Carbonyl, Thiocarbonyl, Sulfonamido, Carboxy, Sulfinyl, Sulfonyl, O-Carbamyl, N-Carbamyl, O-Thiocarbamyl, N-Thiocarbamyl, C-Amido, Amino und -NR¹⁸R¹⁹, mit R¹⁸ und R¹⁹ wie oben definiert, substituiert ist;
Heteroalicyclen aus der Gruppe, die aus Piperidin, Morpholin, Piperazin, Pyrrolin, Pyrrolidin, Dihydrothiophen und Tetrahydrofuran besteht, ausgewählt werden, und optional mit unsubstituiertem C1-C4 Alkyl; unsubstituiertem Cycloalkyl wie oben beschrieben; Halogen; Trihalomethyl; Hydroxy; C1-C4 Alkoxy; Aryloxy; Thiohydroxy; C1-C4 Thioalkoxy; Thioaryloxy; Cyano; Nitro; Carbonyl; Thiocarbonyl; Carboxy; O-Carbamyl; N-Carbamyl; O-Thiocarbamyl; N-Thiocarbamyl; Sulfinyl; Sulfonyl; C-Amido; N-Amido; Amino und -NR¹⁸R¹⁹, mit R¹⁸ und R¹⁹ wie oben definiert, substituiert sind.

2. Die Verbindung oder das Salz nach Anspruch 1, wobei R¹ Wasserstoff ist.

3. Die Verbindung oder das Salz nach Anspruch 1, wobei R² Wasserstoff ist.

4. Die Verbindung oder das Salz nach Anspruch 1, wobei R⁷ Wasserstoff ist.

5. Die Verbindung oder das Salz nach einem der Ansprüche 1 bis 4, wobei R³, R⁴, R⁵ und R⁶ unabhängig voneinander ausgewählt werden aus der Gruppe, die besteht aus:
Wasserstoff;
unsubstituiertem C1-C4 Alkyl;
C1-C4 Alkyl substituiert mit einer Gruppe, die aus der Gruppe, die aus unsubstituiertem Cycloalkyl, Halogen, Hydroxy, unsubstituiertem C1-C4 Alkoxy, C-Carboxy, unsubstituiertem Aryl, unsubstituiertem Heteroaryl, unsubstituiertem Heteroalicyclen, Amino, quaternärem Ammonium oder -NR¹⁸NR¹⁹ besteht, ausgewählt wird;
unsubstituiertem Cykloalkyl;
Hydroxy;
unsubstituiertem C1-C4 Alkoxy;
C1-C4 Alkoxy substituiert mit einer Gruppe, die aus der Gruppe, die aus einer oder mehreren Halogengruppen, unsubstituiertem Aryl oder unsubstituiertem Heteroaryl besteht, ausgewählt wird;
Trihalogenmethyl;
Halogen;
unsubstituiertem Aryl;
Aryl substituiert mit einer oder mehreren Gruppen, die unabhängig aus der Gruppe, die aus unsubstituiertem C1-C4 Alkyl, C1-C4 Alkyl substituiert mit einer oder mehr Halogengruppen, Trihalogenmethyl, Halogen, Hydroxy oder unsubstituiertem C1-C4 Alkoxy besteht, ausgewählt wird;
unsubstituiertem Aryloxy;
Aryloxy substituiert mit einer oder mehreren Gruppen, die unabhängig voneinander aus der Gruppe, die aus Halogen, unsubstituiertem C1-C4 Alkyl, C1-C4 Alkyl substituiert mit einer oder mehr Halogengruppen, unsubstituiertem Aryl, Hydroxy, unsubstituiertem C1-C4 Alkoxy, Amino- oder -NR¹⁸NR¹⁹ besteht, ausgewählt werden;
S-Sulfonamido;
unsubstituiertem Heteroaryl;
Heteroaryl substituiert mit einer oder mehreren Gruppen, die unabhängig aus der Gruppe, die aus Wasserstoff, unsubstituiertem C1-C4 Alkyl, C1-C4 Alkyl substituiert mit einer oder mehreren Halogengruppen, Trihalogenmethyl, Halogen, Hydroxy, unsubstituiertem C1-C4 Alkoxy, unsubstituiertem Aryloxy, Amino, S-Sulfonamido oder-NR¹⁸R¹⁹ besteht, ausgewählt werden;
unsubstituierten Heteroalicyclen;
Heteroalicyclen substituiert mit einer oder mehreren Gruppen, die unabhängig aus der Gruppe, die aus unsubstituiertem C1-C4 Alkyl, C1-C4 Alkyl substituiert mit einer oder mehreren Halogengruppen, unsubstituiertem C1-C4 Alkyl, Alkoxy, Hydroxy, Amino, Amino, S-Sulfonamido, oder -NR¹⁸R¹⁹ besteht, ausgewählt werden;
unsubstituiertem C1-C4 Alkyl C-Carboxy;
Karbonsäure;
unsubstituiertem C1-C4 Alkylcarbonyl;
Aldehyd;
unsubstituiertem Arylcarbonyl;
Acetyl;
S-Sulfonamido;
N-Sulfonamido;
Amino; und
NR¹⁸NR¹⁹.

6. Die Verbindung oder das Salz nach einem der Ansprüche 1 bis 5, wobei zwei beliebige von R⁸, R^{8'} oder R^{8''} -(alk₁)rM Gruppen sind.

7. Die Verbindung oder das Salz nach einem der Ansprüche 1 bis 6, wobei J¹ aus der Gruppe, die aus Stickstoff, Sauerstoff und Schwefel besteht, ausgewählt wird und J², J³ und J⁴ Kohlenstoff sind.

8. Die Verbindung oder das Salz nach einem der Ansprüche 1 bis 6, wobei J¹ und J³ Stickstoff und J² und J⁴ Kohlenstoff sind.

9. Eine pharmazeutische Zusammensetzung, die eine Verbindung oder ein Salz gemäß einem der Ansprüche 1 bis 8 und einen pharmazeutisch annehmbaren Träger oder Verdünnungsmittel umfasst.

10. Die Verwendung einer Verbindung gemäß einem der Ansprüche 1 bis 8 zur Herstellung eines Medikamentes für die Behandlung einer Krankheit, die aus einer Krebserkrankung ausgewählt wird, die aus der Gruppe, die aus Plattenepithelkarzinom, Astrocytom, Glioblastom, Brustkrebs, Gliom, Melanom, Lungenkrebs, kleinzelliger Lungenkrebs, Blasenkrebs, Eierstockkrebs, Prostatakrebs und Kopf- und Halskrebs besteht; einer Proliferationsstörung, die aus der Gruppe, die aus Restinose, Fibrose, Psoriasis, Osteoarthritis und rheumatoide Arthritis, Diabetes, Autoimmunkrankheiten, einer entzündlichen Fehlfunktion und Angiogenese besteht, ausgewählt wird.

## Revendications

1. 2-indolone ayant la structure chimique : 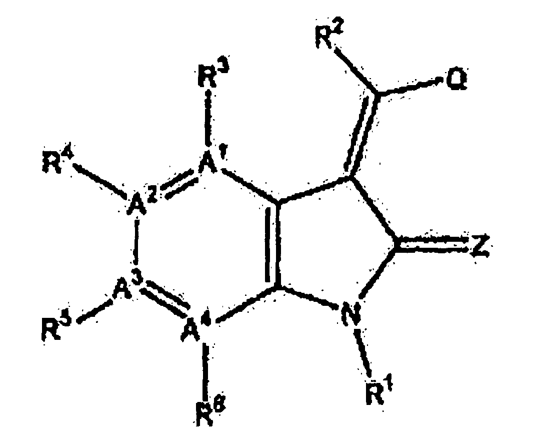 dans laquelle :
A² et A⁴ sont l'azote et A¹ et A³ sont le carbone ou A¹ et A³ sont l'azote et A² et A⁴ sont le carbone, étant entendu que lorsque A¹, A², A³ et A⁴ sont l'azote, R³, R⁴, R⁵ et R⁶, respectivement n'existent pas ;
R¹ est choisi dans le groupe constitué par l'hydrogène, un groupe alkyle en C1-C10, cycloalkyle, alcényle en C2-C10, alkynyle en C2-C10,. aryle, hétéroaryle, trihalométhanecarbonyle, hétéroalicyclique, hydroxy, alcoxy en C1-C10, C-carboxy, O-carboxy, C-amido, C-thioamido, guanyle, guanadinyle, uréidyle, sulfonyle et trihalométhanesulfonyle ;
R² est choisi dans le groupe constitué par l'hydrogène, un groupe alkyle en C1-C10, cycloalkyle, aryle et un groupe halo ;
R³, R⁴, R⁵ et R⁶ sont indépendamment choisis dans le groupe constitué par l'hydrogène, un groupe alkyle en C1-C10, trihalométhyle, cycloalkyle, alcényle en C2-C10, alkynyle en C2-C10, aryle, hétéroaryle, hétéroalicyclique, hydroxy, thiohydroxy, alcoxy en C1-C10, aryloxy, thiohydroxy, thioalcoxy en C1-C10, thioaryloxy, sulfinyle, sulfonyle, S-sulfonamido, N-sulfonamido, N-trihalométhanesulfonamido, carbonyle, aldéhyde, trihalométhyl-carbonyle, C-carboxy, O-carboxy, cyano, nitro, halo, cyanato, isocyanato, thiocyanato, isothiocyanato, O-carbamyle, N-carbamyle, O-thiocarbamyle, N-thiocarbamyle, phosphonyle, guanyle, guanidinyle, uréidyle, C-amido, N-amido, amino, ammonium quaternaire, -NR¹⁸R¹⁹ ;
R¹⁸ et R¹⁹ sont indépendamment choisis dans le groupe constitué par l'hydrogène, un groupe alkyle en C1-C10, cycloalkyle, aryle, carbonyle, acétyle, trihalométhylcarbonyle, C-carboxy, trihalométhanesulfonyle, sulfonyle, C-peptidyle et, combinés, un cycle hétéroalicylique à cinq ou six chaînons ;
Z est l'oxygène ;
Q est 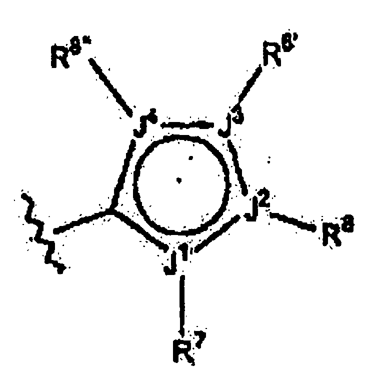
J¹ est choisi dans le groupe constitué par l'oxygène, l'azote et le soufre ;
J², J³ et J⁴ sont indépendamment choisis dans le groupe constitué par le carbone, l'azote, l'oxygène et le soufre de sorte que le cycle hétéroaryle à cinq chaînons est un cycle connu dans l'art de la chimie, étant, en outre, entendu que lorsque J², J³ ou J⁴ est l'azote, l'oxygène ou le soufre, R⁸, R^{8'} ou R^{8''} respectivement, n'existent pas ; de la même manière, lorsque J¹ est l'oxygène ou le soufre, R⁷ n'existe pas ;
R⁷ est choisi dans le groupe constitué par l'hydrogène, un groupe alkyle en C1-C10, cycloalkyle, trihalométhylcarbonyle, aryle, guanyle, carboxyle, sulfonyle, et trihalométhanesulfonyle ;
l'un de R⁸, R^{8'} ou R^{8''} est H ou un groupe -(alk₁) rM ; dans lequel (alk₁) est CH₂ ; r vaut de 1 à 3 inclus ; et M est choisi dans le groupe constitué par un groupe hydroxy, amino, carboxy, morpholinyle, pipérazinyle, tétrazolyle, N-carbamyle, O-carbamyle, S-sulfonamido, N-sulfonamido, sulfonyle, -S(O)₂OH et phosphonyle ;
les deux restants de R⁸, R⁸' et R⁸" sont indépendamment choisis dans le groupe constitué par :
l'hydrogène ;
un groupe alkyle en C1-C4 non substitué ;
un groupe alkyle en C1-C4 substitué par un ou plusieurs groupes choisis dans le groupe constitué par les groupes : halo, hydroxyle, alcoxy en C1-C4 non substitué, amino, S-sulfonamido, -NR¹⁸R¹⁹ ou carboxy ;
un groupe alcoxy en C1-C4 non substitué ;
un groupe alcoxy en C1-C4 substitué par un ou plusieurs halogènes ;
un groupe aryle non substitué ;
un groupe aryle substitué par un ou plusieurs groupes choisis dans le groupe constitué par un groupe alkyle en C1-C4 non substitué, alcoxy en C1-C4 non substitué, halo, trihalométhyle, amino, -NR¹⁸R¹⁹, hydroxy ou S-sulfonamido ;
un groupe hétéroaryle non substitué ;
un groupe hétéroaryle substitué par un ou plusieurs groupes choisis parmi les groupes alkyle en C1-C4 non substitué, alcoxy en C1-C4 non substitué, halo, trihalométhyle, amino, -NR¹⁸R¹⁹, hydroxy ou S-sulfonamido ;
un groupe hétéroalicyclique non substitué ;
un groupe hétéroalicyclique substitué par un ou plusieurs groupes choisis parmi les groupes alkyle en C1-C4 non substitué, halo, hydroxy, alcoxy non substitué, amino ou -NR¹⁸R¹⁹ ;
un groupe halo ;
un groupe cyano ;
un groupe carboxy ; et
un cycle cycloalkyle, aryle, hétéroaryle ou hétéroalicyclique à six chaînons formé en combinant R⁸ et R⁸' ou R⁸' et R⁸" ;
et leurs sels physiologiquement acceptables ;
et dans laquelle :
un groupe alkyle en C1-C10, est éventuellement substitué par un groupe cycloalkyle non substitué choisi dans le groupe constitué par le cyclopropane, le cyclobutane, le cyclopentane, le cyclopentène, le cyclohexane, l'adamantane, le cyclohexadiène, le cycloheptane et le cycloheptatriène ; un groupe aryle non substitué choisi dans le groupe constitué par le phényle, le naphthalényle et l'anthracényle ; un groupe hétéroaryle non substitué choisi dans le groupe constitué par le pyrrole, le furane, le thiophène, l'imidazole, l'oxazole, le thiazole, le pyrazole, la pyridine, la pyrimidine, la quinoline, l'isoquinoline, la purine et le carbazole ; un groupe hétéroalicyclique non substitué choisi dans le groupe constitué par la pipéridine, la morpholine, la pipérazine, la pyrroline, la pyrrolidine, le dihydrothiophène et le tétrahydrofurane ; un groupe hydroxy ; un groupe alcoxy en C1-C4, un groupe aryloxy ; un groupe thiohydroxy ; un groupe thioalcoxy en C1-C4 : un groupe thioaryloxy ; un groupe cyano ; un groupe halo ; un groupe carbonyle ; un groupe thiocarbonyle ; un groupe O-carbamyle ; un groupe N-carbamyle ; un groupe O-thiocarbamyle; un groupe N-thiocarbamyle ; un groupe C-amido ; un groupe N-amido ; un groupe C-carboxy ; un groupe O-carboxy; un groupe cyanato ; un groupe isocyanato ; un groupe thiocyanato ;, un groupe isothiocyanato ; un groupe nitro ; un groupe silyle ; un groupe amino et un groupe -NR¹⁸R¹⁹, où R¹⁸ et R¹⁹ sont indépendamment choisis dans le groupe constitué par l'hydrogène, un groupe alkyle en C1-C4 non substitué, un groupe cycloalkyle non substitué tel que défini ci-dessus, un groupe aryle non substitué tel que défini ci-dessus, un groupe carbonyle, un groupe trihalométhylcarbonyle, un groupe C-carboxy, un groupe trihalométhylsulfonyle, un groupe sulfonyle et, combiné, un cycle hétéroalicyclique à cinq ou six chaînons tel que défini ci-dessus ;
un groupe cycloalkyle est choisi dans le groupe constitué par le cyclopropane, le cyclobutane, le cyclopentane, le cyclopentène, le cyclohexane, l'adamantane, le cyclohexadiène, le cycloheptane et le cycloheptatriène, et est éventuellement substitué par un groupe alkyle en C1-C4 non substitué ; un groupe aryle non substitué tel que défini ci-dessus ; un groupe hétéroaryle non substitué tel que défini ci-dessus ; un groupe hétéroalicyclique non substitué tel que défini ci-dessus ; un groupe hydroxy ; un groupe alcoxy en C1-C4 ; un groupe aryloxy ; un groupe thiohydroxy ; un groupe thioalcoxy en C1-C4 ; un groupe thioaryloxy ; un groupe cyano ; un groupe halo ; un groupe carbonyle ; un groupe thiocarbonyle ; un groupe carboxy ; un groupe O-carbamyle ; un groupe N-carbamyle ; un groupe C-amido ; un groupe N-amido ; un groupe nitro ; un groupe amino et un groupe -NR¹⁸R¹⁹, avec R¹⁸ et R¹⁹ tels que définis ci-dessus ;
un groupe alcényle en C2-C10 est éventuellement substitué tel que défini pour le groupe alkyle en C1-C10 ci-dessus ;
un groupe alkynyle en C2-C10 est éventuellement substitué tel que défini précédemment pour le groupe alkyle en C1-C10 ci-dessus ;
un groupe aryle est choisi dans le groupe constitué par le phényle, le naphthalényle et l'anthracényle, et est éventuellement substitué par un groupe halo ; un groupe trihalométhyle ; un groupe alkyle en C1-C4 non substitué ; un groupe aryle non substitué tel que défini ci-dessus ; un groupe hétéroaryle non substitué tel que défini ci-dessus ; un groupe hétéroalicyclique non substitué tel que défini ci-dessus ; un groupe hydroxy ; un groupe alcoxy en C1-C4 ; un groupe aryloxy ; un groupe thiohydroxy ; un groupe thioalcoxy en C1-C4 ; un groupe thioaryloxy ; un groupe cyano ; un groupe nitro ; un groupe carbonyle ; un groupe thiocarbonyle ; un groupe C-carboxy ; un groupe O-carboxy; un groupe O-carbamyle ; un groupe N-carbamyle ; un groupe O-thiocarbamyle ; un groupe N-thiocarbamyle ; un groupe C-amido ; un groupe N-amido ; un groupe sulfinyle ; un groupe sulfonyle ; un groupe S-sulfonamido ; un groupe N-sulfonamido ; un groupe trihalométhanesulfonamido ; un groupe amino et un groupe -NR¹⁸R¹⁹, avec R¹⁸ et R¹⁹ tels que définis ci-dessus ;
un groupe hétéroaryle est choisi dans le groupe constitué par le pyrrole, le furane, le thiophène, l'imidazole, l'oxazole, le thiazole, le pyrazole, la pyridine, la pyrimidine, la quinoline, l'isoquinoline, la purine et le carbazole, et est éventuellement substitué par un groupe alkyle en C1-C4 non substitué ; un groupe cycloalkyle non substitué tel que défini ci-dessus ; un groupe aryle non substitué tel que défini ci-dessus ; un groupe halo ; un groupe trihalométhyle ; un groupe hydroxy ; un groupe alcoxy en C1-C4, un groupe aryloxy ; un groupe thiohydroxy ; un groupe thioalcoxy en C1-C4 ; un groupe thioaryloxy ; un groupe cyano ; un groupe nitro ; un groupe carbonyle ; un groupe thiocarbonyle ; un groupe sulfonamido ; un groupe carboxy ; un groupe sulfinyle ; un groupe sulfonyle ; un groupe O-carbamyle ; un groupe N-carbamyle ; un groupe O-thiocarbamyle ; un groupe N-thiocarbamyle ; un groupe C-amido ; un groupe N-amido ; un groupe amino et un groupe -NR¹⁸R¹⁹, avec R¹⁸ et R¹⁹ tels que définis ci-dessus ;
un groupe hétéroalicyclique est choisi dans le groupe constitué par la pipéridine, la morpholine, la pipérazine, la pyrroline, la pyrrolidine, le dihydrothiophène et le tétrahydrofurane, et est éventuellement substitué par un groupe alkyle en C1-C4 non substitué ; un groupe cycloalkyle non substitué tel que défini ci-dessus ; un groupe halo ; un groupe trihalométhyle ; un groupe hydroxy ; un groupe alcoxy en C1-C4 ; un groupe aryloxy ; un groupe thiohydroxy ; un groupe thioalcoxy en C1-C4 ; un groupe thioaryloxy ; un groupe cyano ; un groupe nitro ; un groupe carbonyle ; un groupe thiocarbonyle ; un groupe carboxy ; un groupe O-carbamyle ; un groupe N-carbamyle ; un groupe O-thiocarbamyle ; un groupe N-thiocarbamyle ; un groupe sulfinyle ; un groupe sulfonyle ; un groupe C-amido ; un groupe N-amido ; un groupe amino et un groupe -NR¹⁸R¹⁹, avec R¹⁸ et R¹⁹ tels que définis ci-dessus ;

2. Composé ou sel selon la revendication 1, dans lequel R¹ est l'hydrogène.

3. Composé ou sel selon la revendication 1, dans lequel R² est l'hydrogène.

4. Composé ou sel selon la revendication 1, dans lequel R⁷ est l'hydrogène.

5. Composé ou sel selon l'une quelconque des revendications 1 à 4 dans lequel R³, R⁴, R⁵ et R⁶ sont indépendamment choisis dans le groupe constitué par :
l'hydrogène ;
un groupe alkyle en C1-C4 non substitué
un groupe alkyle en C1-C4 substitué par un groupe choisi dans le groupe constitué par les groupes cycloalkyle non substitué, halo, hydroxy, alcoxy en C1-C4 non substitué, C-carboxy, aryle non substitué, hétéroaryle non substitué, hétéroalicyclique non substitué, amino, ammonium quaternaire ou -NR¹⁸R¹⁹ ;
un groupe cycloalkyle non substitué ;
un groupe hydroxy ;
un groupe alcoxy en C1-C4 non substitué ;
un groupe alcoxy en C1-C4 substitué par un groupe choisi dans le groupe constitué par un ou plusieurs groupes halo, un groupe aryle non substitué ou un groupe hétéroaryle non substitué ;
un groupe trihalométhyle ;
un groupe halo ;
un groupe aryle non substitué ;
un groupe aryle substitué par un ou plusieurs groupes indépendamment choisis dans le groupe constitué par un groupe alkyle en C1-C4 non substitué, un groupe alkyle en C1-C4 substitué par un ou plusieurs groupes halo, un groupe trihalométhyle, un groupe halo, un groupe hydroxy ou un groupe alcoxy en C1-C4 non substitué ;
un groupe aryloxy non substitué ;
un groupe aryloxy substitué par un ou plusieurs groupes indépendamment choisis dans le groupe constitué par un groupe halo, un groupe alkyle en C1-C4 non substitué, un groupe alkyle en C1-C4 substitué par un ou plusieurs groupes halo, un groupe aryle non substitué, un groupe hydroxy, un groupe alcoxy en C1-C4 non substitué, un groupe amino ou -NR¹⁸R¹⁹ ;
un groupe S-sulfonamido ;
un groupe hétéroaryle non substitué ;
un groupe hétéroaryle substitué par un ou plusieurs groupes indépendamment choisis dans le groupe constitué par l'hydrogène, un groupe alkyle en C1-C4 non substitué, un groupe alkyle en C1-C4 substitué par un ou plusieurs groupes halo, un groupe trihalométhyle, un groupe halo, un groupe hydroxy, un groupe alcoxy en C1-C4 non substitué, un groupe aryloxy non substitué, un groupe amino, un groupe S-sulfonamido ou -NR¹⁸R¹⁹ ;
un groupe hétéroalicyclique non substitué ;
un groupe hétéroalicyclique substitué par un ou plusieurs groupes indépendamment choisis dans le groupe constitué par un groupe alkyle en C1-C4 non substitué, un groupe alkyle en C1-C4 substitué par un ou plusieurs groupes halo, un groupe alcoxy alkyle en C1-C4 non substitué, un groupe hydroxy, un groupe amino, un groupe S-sulfonamido ou -NR¹⁸R¹⁹ ;
un groupe C-carboxy-alkyle en C1-C4 non substitué ;
un acide carboxylique ;
un groupe carbonyle-alkyle en C1-C4 non substitué ;
un aldéhyde ;
un groupe carbonyl-aryle non substitué ;
un groupe acétyle ;
un groupe S-sulfonamido ;
un groupe N-sulfonamido ;
un groupe amino ; et,
un groupe -NR¹⁸R¹⁹.

6. Composé ou sel selon l'une quelconque des revendications 1 à 5, dans lequel deux quelconques de R⁸, R^{8'} ou R^{8"} sont des groupes -(alk₁)rM.

7. Composé ou sel selon l'une quelconque des revendications 1 à 6, dans lequel J¹ est choisi dans le groupe constitué par l'azote, l'oxygène et le soufre et J², J³ et J⁴ sont le carbone.

8. Composé ou sel selon l'une quelconque des revendications 1 à 6, dans lequel J¹ et J3 sont l'azote et J² et J⁴ sont le carbone.

9. Composition pharmaceutique comprenant un composé ou sel selon l'une quelconque des revendications 1 à 8 et un porteur ou diluant pharmaceutiquement acceptable.

10. Utilisation d'un composé selon l'une quelconque des revendications 1 à 8 dans la fabrication d'un médicament destiné au traitement d'une maladie choisie parmi les cancers dans le groupe constitué par le carcinome à cellules squameuses, l'astrocytome, le glioblastome, le cancer du sein, le gliome, le mélanome, le cancer du poumon, le cancer du poumon à petites cellules, le cancer de la vessie, le cancer des ovaires, le cancer de la prostate, et le cancer de la tête et du cou ; un trouble de la prolifération choisi dans le groupe constitué par la resténose, la fibrose, le psoriasis, l'ostéoarthrite et la polyarthrite rhumatoïde ; les diabètes, une maladie auto-immune, un trouble inflammatoire et l'angiogénèse.
